# EUROPEAN PATENT APPLICATION

(11) **EP 4 070 814 A1**
(43) Date of publication of application: **12.10.2022**
(21) Application number: 21305445.5
(22) Date of filing: 07.04.2021
(51) Int. Cl.: A61K 39/12, C07K 16/10, A61P 31/14, C07K 14/005, G01N 33/569

(54) **SARS-COV-2 POLYPEPTIDES AND USES THEREOF**

(71) Applicant: Lama France, 75001 Paris (FR)
(72) Inventor: DEBRE, Patrice, 75006 PARIS (FR); VIEILLARD, Vincent, 75004 PARIS (FR); PRADEYROL, Christian, 75001 PARIS (FR)
(74) Representative: Nony

(57) **Abstract**

The present disclosure relates to a HRl-derived polypeptide comprising 10 or more consecutive amino acids of the polypeptide of SEQ ID NO. 8 as disclosed in the description, or a polypeptide derivative thereof.

This disclosure also concerns an immunogenic composition comprising a HRl-derived polypeptide as described herein.

It also relates to an immunogenic composition comprising a nucleic acid encoding a HRl-derived polypeptide as described herein. In some preferred embodiments, the said nucleic acid consists of a ribonucleic acid.

## Description

### FIELD OF THE DISCLOSURE

This disclosure relates to the field of the therapeutic treatment of human subjects infected with a SARS-COV-2 virus.

### BACKGROUND OF THE DISCLOSURE

SARS-COV-2 is an enveloped, positive-sense, single-stranded RNA virus that targets the cell via the combination of viral structural spike protein (S protein) and the angiotensin-converting enzyme 2 (ACE2) receptor on the host cell. Infection with SARS-COV-2 has spread all over the world and become a global pandemic, this virus appearing to be relatively mild with a low lethality as compared to SARS or MERS viruses, but far more contagious.

Since the first genome sequence of SARS-COV-2 was released, several structures have been reported for S protein complexes, including the ectodomain stabilized in the prefusion conformation and RBD-ACE2 complexes, building upon the previous success of the structural biology of S proteins from other COVs. In the stabilized S ectodomain, The S protein is a heavily glycosylated type I membrane protein anchored in the viral membrane. It is first produced as a precursor that trimerizes and is thought to be cleaved by a furin-like protease into two fragments : the receptor-binding fragment (RBD) S1 and the fusion fragment S2. Binding through the RBD in S 1 to a host cell receptor ACE2 and further protein cleavage at a second site in S2 by a serine protease (TMPRSS2) of the endosomal cysteine proteases cathepsins B and L (CatB/L) are believed to trigger dissociation of S1 and irreversible folding of S2 into a post-fusion conformation, a trimeric hairpin structure formed by heptad repeat 1 (HR1) and heptad repeat 2 (HR2). These large structural rearrangements bring together the viral and cellular membranes, ultimately leading to fusion of the two bilayers.

SARS-COV-2 virus particles in the infected cells are recognized as pathogen-associated molecular patterns by soluble pattern recognition receptors. These molecules are further recognized by toll-like receptors present on the immune cells like dendritic cells (DCs), natural killer cells (NKs), neutrophils, macrophages, T cells and B cells, leading to their activation. Following this, these immune cells produce type 1 interferon and other pro-inflammatory cytokines. According to a meta-analysis study, it was revealed that the levels of most cytokines- IL-1 β , IL-1R α , IL-2, IL-2R α, IL-4, IL-5, IL-6, IL-7, IL-8 (CXCL8), IL-9, IL-10, IL-12 (p70), IL-13, IL-15, IL-17, TNF- α, IFN- γ, IP-10 (CXCL10), GCSF, MIP-1 α (CCL3), M-CSF, CTACK, GM-CSF, MCP-1 (CCL2), FGF basic, RANTES (CCL5), and Eotaxinin were elevated in COVID-19 patients when compared to healthy individuals. Among these, IL-1R α, IL-2, IL-2R α, IL-6, IL-8 (CXCL8), IL-10, G-CSF, HGF, MCP-3, and MIG were further elevated in severe infections whereas TNF- α was higher in patients in ICU care (Ramezani et al., 2020, Chemokines in COVID-19 Patients: A Systematic Review and Meta- Analysis. https://doi.org/10.2139/ssrn.3582815, 4/20/2020).

The extent of activation of these immune cells and the production of pro-inflammatory cytokines measures the severity of the disease pathology, a cytokine storm escalation. These cytokines are responsible for symptoms associated with SARS-COV-2 fever, coughing, sneezing, chills, fatigue, pneumonia, hemoptysis, lymphopenia, ground-glass opacities in lungs and, in severe cases, acute respiratory distress syndrome (ARDS) and multiple organ failure which are the cause of most mortalities (Rothan et al., 2020, J Autoimmun, Vol. 109 : 102433).

Given the severity of the disease caused by an infection with SARS-COV-2, there is an urgent need of therapeutics or vaccines to treat or prevent the spread of SARS-COV-2, as well as to treat the deleterious physiological consequences of the viral infection.

On the side of the prevention of the infection, various kinds of vaccines have already been developed and massive vaccination in a plurality of countries have been initiated.

On the side of the antiviral treatments, much like influenza, antiviral drugs likely need to be started early after infection to be effective. In turn, this makes it difficult to identify drugs that are indeed effective against the virus in clinical trials. There have been extensive efforts to repurpose approved antiviral drugs but with a low success to date. Therapeutic antibodies directed to the SARS-COV-2 particles, such as the dual-antibody cocktail termed REGN-COV-2 marketed by Regeneron Company may be cited among the relevant drug candidates. Administration of plasma derived from convalescent SARS-COV-2 subjects has also been tested, but on a too much reduced sample size to be conclusive.

According to another strategy, various drugs aimed at reducing the deleterious consequences of the viral infection are being studied, like (i) dexamethasone for modulating inflammation-mediated lung injury and thereby reduce progression to respiratory failure and death, (ii) Immunosuppressive antibodies such as Tocilizumab and Sarimumab and (iii) kinase inhibitors used for controlling downstream inflammation.

There remains a high need for availability of further prophylactic and therapeutic tools against SARS-COV-2.

### SUMMARY OF THE DISCLOSURE

The present disclosure relates to a HR1-derived polypeptide comprising 10 or more consecutive amino acids of the polypeptide of SEQ ID NO. 8, or a polypeptide derivative thereof.

In some embodiments, the said HR1-derived polypeptide comprises 10 or more consecutive amino acids of a peptide selected in the group consisting of SEQ ID NO. 1 to SEQ ID NO. 5, or a polypeptide derivative thereof.

This disclosure also pertains to a nucleic acid encoding a HR1-polypeptide as described herein, to an expression vector comprising the said nucleic acid and to a recombinant host cell comprising the said nucleic acid.

This disclosure also concerns an immunogenic composition comprising a HR1-derived polypeptide as described herein.

It also relates to an immunogenic composition comprising a nucleic acid encoding a HR1-derived polypeptide as described herein. In some preferred embodiments, the said nucleic acid consists of a ribonucleic acid.

The present disclosure further pertains to an antibody directed against a HR-1-derived polypeptide as described herein, which encompasses a monoclonal antibody. It also pertains to such an antibody for its use for treating a subject infected with a SARS-COV-2 virus.

It also concerns an *in vitro* method for detecting and/or quantifying antibodies directed against a HR1-derived polypeptide as described herein in a sample, comprising the following steps:
a) bringing into contact a sample to be tested with one or more HR1-derived polypeptide as described herein, and
b) detecting and/or quantifying the formation of complexes between the said HR1-derived polypeptide and antibodies present in the said sample.

This disclosure pertains to an *in vitro* method for the screening of compounds useful for preventing a disease caused by the infection of a subject with a SARS-COV-2 virus comprising the steps of :
a) bringing into contact cultured cells in which NKp44L membrane expression is inducible with a candidate compound,
b) adding a HR1-derived peptide of the disclosure to the cultured cells obtained at step a),
c) determining NKp44L membrane expression by the cultured cells obtained at step b), and
d) selecting the candidate compound when NKp44L membrane expression is lower than the NKp44L membrane expression that is determined when step a) is performed in the absence of the said candidate compound.

This disclosure also concerns an *in vitro* method for the screening of compounds useful for treating a disease caused by the infection of a subject with a SARS-COV-2 virus comprising the steps of :
a) bringing into contact cultured cells in which NKp44L membrane expression is inducible with a HR1-derived peptide of the disclosure,
b) adding a candidate compound to the cultured cells obtained at step a),
c) determining NKp44L membrane expression by the cultured cells obtained at step b), and
d) selecting the candidate compound when NKp44L membrane expression is lower than the NKp44L membrane expression that is determined when step a) is performed in the absence of the said candidate compound.

This disclosure also relates to an *in vitro* method for the screening of compounds useful for preventing and/or treating a disease caused by the infection of a subject with a SARS-COV-2 virus comprising the steps of :
a) bringing into contact cultured cells in which NKp44L membrane expression is inducible with (i) a HR1-derived peptide of the disclosure and (ii) a candidate compound,
b) determining NKp44L membrane expression by the cultured cells obtained at step a), and
c) selecting the candidate compound when NKp44L membrane expression is lower than the NKp44L membrane expression that is determined when step a) is performed in the absence of the said candidate compound.

### DETAILED DESCRIPTION OF THE DISCLOSURE

The present disclosure relates to prophylactic and therapeutic tools aimed at treating human subjects infected with a SARS-COV-2 virus.

The inventors have unexpectedly shown that peripheral blood mononuclear cells originating from human subjects infected with SARS-COV-2 express NKp44L at their membrane, NKp44L being a ligand of the NKp44 receptor which is mainly expressed by the Natural Killer (NK) cells. It has been shown that membrane expression of NKp44L is found in SARS-COV-2-infected subjects, particularly in lymphocyte cells having the respective phenotypes of CD3⁺CD8⁺ and CD3⁺CD56⁺. It has been shown that cell-surface NKp44L expression on T CD3⁺CD8⁺ cells and T CD3⁺CD56⁺ cells is found in the hospitalized SARS-COV-2-infected subjects, irrespective of whether these were admitted in intensive care units or not, *i.e.* irrespective of the severity of the disease.

The inventors have also shown that SARS-COV-2 virus particles are themselves endowed with the property of inducing membrane expression of NKp44L by cells, including by cells originating from the human lungs (Calu-3 and ACE-2-tranfected A549). Further, NKp44L membrane expression is induced both by infectious SARS-COV-2 virus particles and by SARS-COV-2 virus particles that have been inactivated. These results show that NKp44L membrane expression induction is directly linked to the presence of the virus particle proteins, irrespective of whether the infectious property of the virus particles is retained or not.

In the examples herein, it is demonstrated that the causal agent of the induced NKp44L expression is comprised in a small region of the SARS-COV-2 spike protein located in the S2 subunit thereof. More precisely, it has been shown that the said causal agent is comprised in the heptad repeat 1 (HR1) contained in the S2 subunit of the SARS-COV-2 spike protein.

As it is known in the art, NKp44 receptor belong to the Natural Cytotoxicity Receptors (NCR) that play an important role in most functions exerted by NK cells. Importantly, NKp44 is only expressed on activated NK cells, in contrast to other NCR, and is implicated in the killing of virus-infected or tumor cells. Published experimental data support an important role of NKp44 in tissue homeostasis and immune regulation. Illustratively, resident NK cells from the liver highly expressed NKp44 in patients chronically infected by hepatitis C virus (HCV); and its frequency of expression is correlated with viral load and fibrosis level. Furthermore, lymphoid tissues associated with epithelia include NKp44⁺ Innate Lymphoid Cells (ILCs) (Freud et al., 2017, Immunity, Vol. 47 : 820-833; Bjorkstrom et al., 2016, Immunology, Vol. 16 : 310-320; Carrega et al., 2015, Nat. Commun., Vol. 6 : 8280; Nel et al., Medicine, Vol. 95: e3678).

As it is also known in the art, NKp44 interacts with a ligand termed NKp44L, the occurrence of the interaction between NKp44 and NKp44L leading to NK cell function.

As used herein, the term "NKp44L" means a peculiar isoform of Mixed-Lineage Leukemia protein-5 (MLL5), which is termed "21spe-MLL5" in the art, which isoform is encoded by a splice variant lacking the last five exons of *MLL5* and containing a unique C-terminal exon, required for both localization at the cell surface and interaction with NKp44. 21spe-MLL5 is mainly found in the cytoplasm and at the cell surface, is barely expressed in healthy tissues but is frequently detected at high levels in hematopoietic and non-hematopoietic tumors cells (Baychelier et al., 2013, Blood, Vol. 122 : 2935-2942). The first evidence of an activating cellular NKp44L was provided in 2005, when it was reported that an NKp44L is induced on CD4⁺ T lymphocytes from HIV-1-infected individuals. It was reported that a fraction of CD4⁺ T cells from HIV-infected patients, but not from healthy subjects, showed an increased expression of NKp44L. The percentage of NKp44L⁺ CD4⁺ T cells was inversely correlated with the CD4⁺ T cell count and correlated with the viral load, suggesting that NKp44L expression may be implicated in T cell depletion and disease course (Vieillard et al., 2005, Proc Natl Acad Sci, Vol. 102: 10981-10986). Generally, upregulation of NKp44L surface expression has been recognized as a response to cellular stress, including stress of cells of the airway epithelium, perhaps representing a component of the pulmonary defense system (Vieillard et al., 2014, Oncoimmunology, Vol. 3, e27988). For the amino acid sequence of human NKp44L, it may be referred to the sequence from the GenBank database n° [JQ809698.1]

Altogether, the results contained in the examples herein show that SARS-COV-2 virus particles are the causal agent of NKp44L expression by cells of the immune system, as well as cells from other tissues, such as cells from the lung tissue, and shall contribute to various deleterious physiological effects encountered in SARS-COV-2-infected subjects.

These results led the inventors to conceiving prophylactic and therapeutic tools aimed at preventing the consequences of NKp44L membrane expression in SARS-COV-2-infected subjects, or alternatively preventing NKp44L membrane expression in SARS-COV-2-infected subjects or further alternatively blocking NKp44L membrane expression in SARS-COV-2-infected subjects.

In some aspects of the present disclosure, preventing the consequences of NKp44L expression may be performed by using agents that prevent the interaction between NKp44 and NKp44L, that is by preventing interaction of cells expressing NKp44, mainly NK cells, with cells expressing NKp44L in subjects infected with SARS-COV-2 virus.

Preventing the consequences of NKp44L expression in subjects infected with SARS-COV-2 virus may be performed by the use of (i) antibodies directed to NKp44 and/or (ii) by antibodies directed to NKp44L. Both (i) antibodies directed to NKp44 and/or (ii) by antibodies directed to NKp44L are known in the art.

According to the most preferred aspects of the present disclosure, preventing NKp44L membrane expression in SARS-COV-2-infected subjects is sought. For reaching this aim, it is taken benefit from the inventor's identification of the SARS-COV-2 agent that causes induction of the expression of NKp44L at the membrane of various cells, including a plurality of lymphocyte T cells and cells from other tissues, such as cells originating from the lung.

### Definitions

The below definitions of terms or expression that are used throughout the present disclosure are aimed at providing an unequivocal meaning to those terms and expressions. The meaning of some of these terms and expressions is illustrated by embodiments that are encompassed by these terms and expressions. These illustrated embodiments describe technical features that are fully part of the corresponding embodiments of the disclosure that are found in the general description, *e.g.* the various technical features with which may be endowed a polypeptide derivative or a peptide derivative according to the present disclosure.

As used herein, a "polypeptide" or a "peptide" is, unless express specification to the contrary, a polypeptide or a peptide that is essentially free from contaminating cellular components, such as carbohydrate, lipid, or other proteinaceous impurities which are generally associated with a polypeptide or peptide in nature. Typically, a preparation of a polypeptide (or a peptide) contains the polypeptide (or the peptide) in a highly purified form, *i.e.*, at least about 80% pure, at least about 90% pure, at least about 95% pure, greater than 95% pure, or greater than 99% pure. One way to show that a particular protein preparation contains a polypeptide at the required degree of purity is by the appearance of a single band following sodium dodecyl sulfate (SDS)-polyacrylamide gel electrophoresis of the protein preparation and Coomassie Brilliant Blue staining of the gel. By definition, polypeptides or peptides, according to their meaning herein, are non-naturally occurring, *i.e.* consist of synthetic polypeptides or peptides, which encompass polypeptides or peptides obtained by chemical synthesis or by genetic engineering methods (*i.e.* recombinant polypeptides or peptides). Methods for isolating or synthesizing peptides of interest with known amino acid sequences are well known in the art.

Polypeptides and peptides of the disclosure are composed of an amino acid chain and may be characterized by their amino acid sequence. Unless otherwise expressly indicated, polypeptides and peptides of this disclosure comprise exclusively natural amino acid residues, which amino acid residues are linked, one to another adjacent amino acid residue by natural peptide bonds.

An amino acid residue comprises an amino terminal part (NH2) and a carboxy terminal part (COOH) separated by a central part (R group) comprising a carbon atom, or a chain of carbon atoms, at least one of which comprises at least one side chain or functional group. NH2 refers to the amino group present at the amino terminal end of an amino acid or peptide, and COOH refers to the carboxy group present at the carboxy terminal end of an amino acid or peptide.

However, this disclosure also encompasses polypeptide or peptide "derivatives", which "derivatives" comprise (i) one or more non-natural amino acid residue(s) and/or (ii) one or more non-natural bonds linking an amino acid residue to an adjacent amino acid residue. Natural amino acid residues are in L-conformation.

Thus, as used herein, "amino acid" or an "amino acid residue" can be a natural or non-natural amino acid residue linked by peptide bonds or bonds distinct from peptide bonds, depending whether the corresponding polypeptide or peptide consists or does not consist of a "polypeptide derivative" or a "peptide derivative".

In polypeptide derivatives and peptide derivatives of the present disclosure, one or more amino acid residues can be in D- configuration (referred to herein as D-enantiomers).

The present disclosure also relates to derivatives of the described polypeptides and peptides, which polypeptide derivatives or peptide derivatives may comprise both natural and non-natural amino acids. Non-natural amino acids include, but are not limited to, modified amino acid residues, and stereoisomers of D-amino acid residues. In those polypeptide or peptide derivatives, naturally occurring amino acids may be further modified, *e.g.* hydroxyproline, γ-carboxyglutamate, and O-phosphoserine.

Thus, polypeptide derivatives or peptide derivatives of the polypeptides or peptides of the disclosure may comprise both natural or non -natural amino acid residues, or any combination thereof.

In some embodiments of polypeptide derivatives or peptide derivatives of the polypeptides or peptides described herein, amino acids may be amino acid analogs or amino acid mimetics. Amino acid analogs refer to compounds that have the same fundamental chemical structure as naturally occurring amino acids, but modified R groups or modified peptide backbones, *e.g.* homoserine, norleucine, methionine sulfoxide, methionine methyl sulfonium. Amino acid mimetics refers to chemical compounds that have a structure that is different from the general chemical structure of an amino acid, but that function in a manner similar. Amino acids may be referred to herein by either their commonly known three letter symbols or by the one-letter symbols recommended by the IUPAC- IUB Biochemical Nomenclature Commission.

Non-natural amino acids may be selected in the group comprising β-alanine (β-Ala) and other omega-amino acids such as 3- aminopropionic acid, 2,3- diaminopropionic acid (Dpr), 4-aminobutyric acid and so forth; - aminoisobutyric acid (Aib); ε-aminohexanoic acid (Aha); δ-aminovaleric acid (Ava); N- methylglycine or sarcosine (MeGIy); ornithine (Orn); citrulline (Cit); t-butylalanine (t-BuA); t-butylglycine (t-BuG); N-methylisoleucine (Melle); phenylglycine (Phg); cyclohexylala ine (Cha); norleucine (NIe); naphthylalanine (Nal); 4- chlorophenylalanine (Phe(4-CJ)); 2∼ fluorophenylalanine (Phe(2-F)); 3-fluorophenylalanine (Phe(3- F)); 4-fluorophenylalanine (Phe(4-F)); penicillamine (Pen); 1,2,3,4-tetrahydroisoquinoiine-3 - carboxylic acid (Tic); β-2- thienylalanine (Thi); methionine sulfoxide (MSO); homoarginine (liArg); N-acetyl lysine (AcLys); 2,4-diaminobutyric acid (Dbu); 2,4-diaminobutyric acid (Dab); p- aminophenylalanine (Phe(pNH.sub.2)); N-methyl valine (MeVal); homocysteine (hCys), homophenylalanine (hPhe) and homoserine (hSer); hydroxyproJine (Hyp), homoproline (hPro), N- methylated amino acids (e.g., N-substituted glycine).

Further, polypeptide derivatives or peptide derivatives of polypeptides or peptides of the present disclosure may comprise "equivalent amino acid residues". This term refers to an amino acid residue capable of replacing another amino acid residue in a polypeptide without substantially altering the structure and/or functionality of the polypeptide. Equivalent amino acids thus have similar properties such as bulkiness of the side chain, side chain polarity (polar or non-polar), hydrophobicity (hydrophobic or hydrophilic), pH (acidic, neutral or basic) and side chain organization of carbon molecules (aromatic/aliphatic). As such, equivalent amino acid residues can be regarded as conservative amino acid substitutions.

In the context of the present disclosure, within the meaning of the term "equivalent amino acid substitution" as applied herein, is meant that in certain embodiments one amino acid may be substituted for another within the groups of amino acids indicated herein below:
i) Amino acids having polar side chains (Asp, Glu, Lys, Arg, His, Asn, Gin, Ser, Thr, Tyr, and Cys);
ii) Amino acids having non-polar side chains (Gly, Ala, Val, Leu, lie, Phe, Trp, Pro, and Met);
iii) Amino acids having aliphatic side chains (Gly, Ala Val, Leu, ile);
iv) Amino acids having cyclic side chains (Phe, Tyr, Trp, His, Pro);
v) Amino acids haying aromatic side chains (Phe, Tyr, Trp);
vi) Amino acids haying acidic side chains (Asp, Glu);
vii) Amino acids having basic side chains (Lys, Arg, His);
viii) Amino acids having amide side chains (Asn, Gin);
ix) Amino acids having hydroxy side chains (Ser, Thr);
x) Amino acids having sulphur-containing side chains (Cys, Met);
xi) Neutral, weakly hydrophobic amino acids (Pro, Ala, Gly, Ser, Thr);
xii) Hydrophilic, acidic amino acids (Gin, Asn, Glu, Asp), and
xiii) Hydrophobic amino acids (Leu, lie, Val).

Still further, polypeptide derivatives or peptide derivatives of polypeptides or peptides of the disclosure may have secondary modifications, such as phosphorylation, acetylation, glycosylation, sulfhydryl bond formation, as long as said modifications retain the functional properties of the original polypeptide or peptide, specifically, the ability to behave as an antigen able, when properly presented to the cells of the immune system in an immunogenic composition, to raise antibodies directed against themselves and most preferably antibodies preventing the induction of NKp44L membrane expression in the presence of SARS-COV-2 virus particles.

The term "nucleic acid" or "nucleic acid sequence" or "nucleotide sequence" refers to polynucleotides including DNA molecules, RNA molecules (including mRNA molecules), cDNA molecules or derivatives. The term encompasses single as well as double stranded polynucleotides. Further, the terms "nucleic acid" and "polynucleotide" are interchangeable and refer to any nucleic acid. The terms "nucleic acid" and "polynucleotide" may also specifically include nucleic acids composed of one or more non-naturally occurring nucleotides, with the nucleobases other than the five biologically occurring bases (adenine, guanine, thymine, cytosine and uracil).

In this connection an "isolated polynucleotide" is a nucleic acid molecule that is separated from the genome of an organism. For example, a mRNA molecule that encodes any of the HR1-derived polypeptides of HR1-derived peptides of the disclosure is an isolated mRNA molecule. Another example of an isolated nucleic acid molecule is a chemically-synthesized nucleic acid molecule that is not integrated in the genome of an organism.

A comparison of sequences and determination of percentage of sequence identity between two sequences can be accomplished using a mathematical algorithm. The skilled person knows several different computer programs that are available to align two sequences and determine the identity between two sequences (Kruskal, J. B. (1983) An overview of sequence comparison In D. Sankoff and J. B. Kruskal, (ed.), Time warps, string edits and macromolecules: the theory and practice of sequence comparison, pp. 1-44 Addison Wesley).

The percent sequence identity between two amino acid sequences or between two nucleic acid sequences may be determined using the Needleman and Wunsch algorithm for the alignment of two sequences. (Needleman, S. B. and Wunsch, C. D. (1970) J. Mol. Biol. 48, 443-453). Both amino acid sequences and nucleotide sequences can be aligned by the algorithm. The Needleman-Wunsch algorithm has been implemented in the computer program NEEDLE.

For the purpose of the disclosure, the NEEDLE program from the EMBOSS package is used (version 2.8.0 or higher, EMBOSS: The European Molecular Biology Open Software Suite (2000) Rice, P. LongdenJ. and Bleasby,A. Trends in Genetics 16, (6) pp276- 277, http://emboss.bioinformatics.nl/). For protein sequences EBLOSUM62 is used for the substitution matrix. For nucleotide sequence, EDNAFULL is used. The optional parameters used are a gap opening penalty of 10 and a gap extension penalty of 0.5. No end gap penalty is added. In the Output section, Yes has been indicated in response to the question "Brief identity and similarity" and "SRS pairwise" indicated as Output alignment format.

After alignment by the program NEEDLE as described above the percentage of sequence identity between a query sequence and a sequence of the disclosure is calculated as follows: Number of corresponding positions in the alignment showing an identical amino acid or identical nucleotide in both sequences divided by the total length of the alignment after subtraction of the total number of gaps in the alignment. The identity defined as herein can be obtained from NEEDLE by using the NOBRIEF option and is labeled in the output of the program as "longest-identity".

The term "nucleic acid vector", refers to a nucleic acid that is used to perform a "carrying" function for another nucleic acid. For example, vectors are often used to allow a nucleic acid to be propagated within a living cell, or to allow a nucleic acid to be packaged for delivery into a cell. A nucleic acid vector, when it refers to a plasmid, refers to cytoplasmic DNA that replicates independently of the bacterial chromosome within a bacterial host cell. In a specific aspect of the present disclosure the term "plasmid" and/or "transfer plasmid" and/or "donor plasmid" refers to an element of recombinant DNA technology useful for construction of *e.g.* recombinant viruses or an expression cassette for insertion into a viral vector.

The term "recombinant" as used herein relates to a RNA nucleic acid, cDNA nucleic acid or protein that does not naturally occur without human intervention. Therefore, the RNA nucleic acid, cDNA nucleic acid or protein is not associated with all or a portion of the sequences with which it is associated in nature.

The term "immunogenic composition" refers to a composition that comprises at least one antigen, which elicits an immunological response in the host to which the immunogenic composition is administered. Such immunological response may be a cellular and/or antibody-mediated immune response to the immunogenic composition of the disclosure. Preferably, the immunogenic composition induces an immune response and, more preferably, confers immunity against one or more of the clinical signs of a SARS-COV-2 infection, and possibly against the SARS-COV-2 virus particles themselves. The host is also described as a "subject". Most preferably, any of the hosts or subjects described or mentioned herein are human individuals.

Usually, an "immunological response" includes but is not limited to one or more of the following effects: the production or activation of antibodies, B cells, helper T cells, suppressor T cells, and/or cytotoxic T cells and/or gamma-delta T cells, directed specifically to an antigen or antigens included in an immunogenic composition of the disclosure.

A "protective immunological response" or "protective immunity" will be demonstrated by either a reduction or lack of clinical signs normally displayed by an infected host, a quicker recovery time and/or a lowered duration of infectivity or lowered pathogen titer in the tissues or body fluids or excretions of the infected host.

The term "pharmaceutical-acceptable carrier" includes any and all solvents, dispersion media, coatings, stabilizing agents, diluents, preservatives, antibacterial and antifungal agents, isotonic agents, adsorption delaying agents, adjuvants, immune stimulants, and combinations thereof.

### Polypeptides according to the disclosure

As it is shown in the examples herein, the inventors have identified an amino acid chain comprised in the HR1 region of the S2 subunit of the SARS-COV-2 spike protein, which amino acid chain comprises a plurality of peptides which individually induce membrane expression of NKp44L. Each of the peptides comprised in the said amino acid chain may thus be a relevant target so as to prevent the cell interaction of the spike protein, or of the virus particle itself, which cell interaction induces NKp44L membrane expression. Other relevant targets disclosed herein are polypeptides comprising one or more of the plurality of HR1-derived peptides identified by the inventors, which polypeptides include a polypeptide comprising all of the HR1-derived peptides identified by the inventors.

It shall be appreciated that none of the polypeptides and peptides described herein can be found *per se* in a natural environment, including in cultures of SARS-COV-2 viral particles nor, according to the applicant's knowledge, in a body fluid or in a body tissue of a subject infected with a SARS-COV-2 virus.

Thus, in preferred embodiments, the polypeptides or peptides described herein are under a form of an isolated polypeptide or peptide, including under a form of a purified polypeptide or peptide.

The individual peptides that are shown in the examples to induce NKp44L membrane expression are the peptides referred to herein as SEQ ID NO. 1, SEQ ID NO. 2, SEQ ID NO. 3, SEQ ID NO. 4 and SEQ ID NO. 5. These peptides may also be termed "HR1-derived peptides" in the present description. These peptides also consist of embodiments of what are termed "HR1-derived polypeptides" throughout the present disclosure.

These individual peptides of SEQ ID NO. 1 to 5 are all comprised in the SARS-COV-2 spike protein HR1-derived polypeptide referred to as SEQ ID NO. 8.

As it will be described hereunder, these polypeptides of interest are preferably used for generating antibodies directed against themselves, with the aim of preventing cell interaction of this HR1 region of the S2 subunit of the SARS-COV-2 spike protein and thus with the aim of preventing or blocking induction of NKp44L membrane expression in subjects infected with the SARS-COV-2 virus. These polypeptides of interest may be collectively termed "HR1-derived polypeptides" in the present description.

The terms "polypeptide" and "peptide" may be used interchangeably throughout the present description, even if the term "peptide" may be used rather for denoting a polypeptide of a short amino acid length.

The present disclosure relates to a HR1-derived polypeptide comprising 10 or more consecutive amino acids of the polypeptide of SEQ ID NO. 8. As already specified herein, each of the HR1-derived peptides of interest (which also consist of HR1-derived polypeptides of the present disclosure) is comprised in the HR1-derived polypeptide of SEQ ID NO. 8.

The peptide of SEQ ID NO. 7 is also termed "Spi 226" herein. The peptide of SEQ ID NO. 1 is also termed "Spi 227" herein. The peptide of SEQ ID NO. 2 is also termed "Spi 229" herein. The peptide of SEQ ID NO. 3 is also termed "Spi 230" herein. The peptide of SEQ ID NO. 4 is also termed "Spi 231" herein. The peptide of SEQ ID NO. 5 is also termed "Spi 232" herein.

The present disclosure also relates to a HR1-derived polypeptide comprising 10 or more consecutive amino acids of a peptide selected in the group consisting of SEQ ID NO. 1 to SEQ ID NO. 5.

As used herein, polypeptides comprising 10 or more consecutive amino acids of a reference peptide selected in the group consisting of SEQ ID NO. 1 to 5 encompass those comprising 11 or more, 12 or more, 13 or more, 14 or more, 15 or more, 16 or more or 17 or more consecutive amino acids of the said reference peptide.

As used herein, polypeptides comprising 10 or more consecutive amino acids of a reference peptide selected in the group consisting of SEQ ID NO. 1 to 5 encompass those comprising 10, 11, 12, 13, 14, 15, 16, 17 or 18 consecutive amino acids of the said reference sequence.

HR1-derived polypeptides of this disclosure encompass each of the peptides selected in the group consisting of SEQ ID NO. 1 to 5.

As it is disclosed in the examples herein, HR1-derived polypeptides encompass the polypeptide of SEQ ID NO. 8, which polypeptide comprises each of the peptides of SEQ ID NO. 1 to 5.

HR1-derived polypeptides of the disclosure encompass polypeptides comprising 10 or more consecutive amino acids of the polypeptide of SEQ ID NO. 8.

As used herein, polypeptides comprising 10 or more consecutive amino acids of SEQ ID NO. 8 encompass those comprising 11 or more, 12 or more, 13 or more, 14 or more, 15 or more, 16 or more, 17 or more, 18 or more, 19 or more, 20 or more, 21 or more, 22 or more, 23 or more, 24 or more, 25 or more, 26 or more, 27 or more, 28 or more, 29 or more, 30 or more, 31 or more, 32 or more, 33 or more, 34 or more, 35 or more, 36 or more, 37 or more, 38 or more, 39 or more, 40 or more and 41 or more consecutive amino acids of the polypeptide of SEQ ID NO. 8.

As used herein, polypeptides comprising 10 or more consecutive amino acids of SEQ ID NO. 7 encompass those comprising 11, 12, 13, 14, 15, 16, 17, 18, 19, 20, 21, 22, 23, 24, 25, 26, 27, 28, 29, 30 31, 32, 33, 34, 35, 36, 37, 38, 39, 40, 41 or 42 consecutive amino acids of the polypeptide of SEQ ID NO. 8.

The present disclosure also pertains to a HR1-derived polypeptide comprising 10 or more consecutive amino acids of the polypeptide of SEQ ID NO. 8, which HR1-derived polypeptide comprises 10 or more consecutive amino acid of a HR1-derived peptide selected in the group of peptides of SEQ ID NO. 1 to SEQ ID NO. 5. As it is readily understood, such a HR1-derived polypeptide may comprise a selected number of consecutive amino acids of more than one HR1-derived peptide selected in the group of peptides of SEQ ID NO. 1 to SEQ ID NO. 5. As it is also readily understood, such a HR1-derived polypeptide may comprise, for two distinct given HR1-derived peptides or part thereof, comprised therein, a distinct number of consecutive amino acids from each of these HR1-derived peptides contained therein. Illustratively, such a HR1-derived polypeptide encompasses a polypeptide comprising (i) 15 consecutive amino acids from the HR1-derived peptide of SEQ ID NO. 1 and (ii) 17 consecutive amino acids of the HR1-derived peptide of SEQ ID NO. 5.

As a reference, the SARS-COV-2 spike protein consists of the polypeptide of the amino acid sequence of SEQ ID NO. 9 described herein.

For the HR1 region of the SARS-COV-2 spike protein, it may be referred to the amino acid sequence of SEQ ID NO. 20. The amino acid sequence of SEQ ID NO. 20 may be encoded by the nucleic acid of SEQ ID NO. 21.

The HR1-derived polypeptide of SEQ ID NO. 8 is comprised in SARS-COV-2 spike protein of SEQ ID NO. 9, Also, the HR1-derived polypeptide of SEQ ID NO. 8 is comprised in the HR1 region of the SARS-COV-2 spike protein of SEQ ID NO. 20.

The amino acid length of a HR1-derived polypeptide according to the present disclosure does not consist of a substantial feature thereof, provided that the other features thereof are met.

Most preferably, the amino acid length of a HR1-derived polypeptide according to the present disclosure may be adapted by the skilled artisan so as to facilitate the use of the said HR1-derived polypeptide as an antigen having the capacity to raise antibodies directed against one or more of the peptides selected in the group consisting of SEQ ID NO. 1 to SEQ ID NO. 5, or more generally antibodies directed to the polypeptide of SEQ ID NO. 8.

Illustratively, the HR1-derived polypeptide of SEQ ID NO. 8 has 42 amino acids in length.

In preferred embodiments, a HR1-derived polypeptide according to this disclosure has up to 100 amino acids in length.

Then, according to these preferred embodiments, a HR1-derived polypeptide of this disclosure has an amino acid length up to 11, 12, 13, 14, 15, 16, 17, 18, 19, 20, 21, 22, 23, 24, 25, 26, 27, 28, 29, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or 100 amino acids in length.

According to these preferred embodiments, a HR1-derived polypeptide has an amino acid length selected in the group of 10, 11, 12, 13, 14, 15, 16, 17, 18, 19, 20, 21, 22, 23, 24, 25, 26, 27, 28, 29, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 and 100 amino acids in length.

In more preferred embodiments, a HR1-derived polypeptide according to the present disclosure has up to 75 amino acids in length, and most preferably up to 50 amino acids in length.

According to these more preferred embodiments, a HR1-derived polypeptide has an amino acid length of up to 10, 11, 12, 13, 14, 15, 16, 17, 18, 19, 20, 21, 22, 23, 24, 25, 26, 27, 28, 29, 29, 30, 31, 32, 33, 34, 35, 36, 37, 38, 39, 40, 41, 42, 43, 44, 45, 46, 47, 48, 49,50, 51, 52, 53, 54, 55, 56, 57, 58, 59, 60, 61, 62, 63, 64, 65, 66, 67, 68, 69, 70, 71, 72, 73, 74 or 75 amino acids in length.

According to these more preferred embodiments, a HR1-derived polypeptide has an amino acid length selected in the group of 10, 11, 12, 13, 14, 15, 16, 17, 18, 19, 20, 21, 22, 23, 24, 25, 26, 27, 28, 29, 29, 30, 31, 32, 33, 34, 35, 36, 37, 38, 39, 40, 41, 42, 43, 44, 45, 46, 47, 48, 49,50, 51, 52, 53, 54, 55, 56, 57, 58, 59, 60, 61, 62, 63, 64, 65, 66, 67, 68, 69, 70, 71, 72, 73, 74 or 75 amino acids in length.

According to these most preferred embodiments, a HR1-derived polypeptide has an amino acid length of up to 10, 11, 12, 13, 14, 15, 16, 17, 18, 19, 20, 21, 22, 23, 24, 25, 26, 27, 28, 29, 29, 30, 31, 32, 33, 34, 35, 36, 37, 38, 39, 40, 41, 42, 43, 44, 45, 46, 47, 48, 49 or 50 amino acids in length.

According to these most preferred embodiments, a HR1-derived polypeptide has an amino acid length selected in the group of 10, 11, 12, 13, 14, 15, 16, 17, 18, 19, 20, 21, 22, 23, 24, 25, 26, 27, 28, 29, 29, 30, 31, 32, 33, 34, 35, 36, 37, 38, 39, 40, 41, 42, 43, 44, 45, 46, 47, 48, 49 and 50 amino acids in length.

In most preferred embodiments, the amino acid sequence of a HR1-derived polypeptide as defined in the present disclosure has at least 90% amino acid identity with the corresponding amino acid sequence found in the spike protein sequence of SEQ ID NO. 9.

As used herein, an amino acid sequence having at least 90% amino acid identity with a reference amino acid sequence encompasses those having at least 91%, 92%, 93%, 94%, 95%, 96%, 97%, 98% or at least 99% amino acid identity with the said reference amino acid sequence.

In some of these most preferred embodiments, the amino acid sequence of a HR1-derived polypeptide as defined in the present disclosure has 100% amino acid identity with the corresponding sequence found in the spike protein sequence of SEQ ID NO. 9.

The present disclosure also pertains to an immunogenic fragment of a HR1-derived polypeptide as described herein, which immunogenic fragment has 10 or more amino acids in length.

As used herein, an "immunogenic fragment" of a HR1-derived polypeptide refers to a fragment of a parent HR1-derived polypeptide that retains the ability to raise an immune response against the said parent HR1-derived polypeptide.

Illustratively, each of the HR1-derived peptides of SEQ ID NO. 1 to SEQ ID NO. 5 consists of an immunogenic fragment of the HR1-derived polypeptide of SEQ ID NO. 8.

The present disclosure also relates to polypeptide derivatives (or peptide derivatives) of the HR1-derived polypeptides (or of the HR1-derived peptides) described herein.

In some embodiments, polypeptide derivatives (or peptide derivatives) comprise one or more amino acid residues in D-configuration.

In some embodiments, polypeptide derivatives (or peptide derivatives) comprise one or more non-peptide bonds linking two consecutive residues.

In some embodiments, polypeptide derivatives (or peptide derivatives) comprise one or more non-natural amino acid residues, such as one or more amino acid analogs or one or more amino acid mimetics.

In some embodiments, polypeptide derivatives (or peptide derivatives) comprise the substitution of one or more amino acid residue contained in a HR1-derived polypeptide (or of a HR1-derived peptide) described herein by an equivalent amino acid.

The present disclosure also relates to a composition comprising one or more HR1-derived polypeptides as described herein.

In accordance with the present disclosure, the term "composition" relates to a composition which comprises at least one of the HR1-derived polypeptides of the present disclosure. A composition of the present disclosure may, optionally and additionally, comprise further compounds such as *e.g.* a pharmaceutically acceptable carrier or further molecules capable of altering the characteristics of a HR1-derived polypeptide described herein thereby, for example, stabilizing, delaying and/or modulating their property, such as its property of raising an immune response leading to preventing or blocking induction of NKp44L membrane expression.

### Nucleic acids

The present disclosure further relates to any nucleic acid encoding any of the HR1-derived polypeptides described herein.

Nucleic acids encoding a HR1-derived polypeptide encompass the following nucleic acids :
- a nucleic acid encoding the HR1-derived polypeptide of SEQ ID NO. 8, which is the nucleic acid of SEQ ID NO. 11,
- a nucleic acid encoding the HR1-derived polypeptide of SEQ ID NO. 1, which is the nucleic acid of SEQ ID NO. 13,
- a nucleic acid encoding the HR1-derived polypeptide of SEQ ID NO. 2, which is the nucleic acid of SEQ ID NO. 15,
- a nucleic acid encoding the HR1-derived polypeptide of SEQ ID NO. 3, which is the nucleic acid of SEQ ID NO. 16,
- a nucleic acid encoding the HR1-derived polypeptide of SEQ ID NO. 4, which is the nucleic acid of SEQ ID NO. 17,
- a nucleic acid encoding the HR1-derived polypeptide of SEQ ID NO. 5, which is the nucleic acid of SEQ ID NO. 18,

Also described herein is the nucleic acid sequence encoding the SARS-COV-2 spike protein of SEQ ID NO. 9; which nucleic acid sequence if of SEQ ID NO. 10.

Also described herein is the nucleic acid sequence encoding the HR1 region of the SARS-COV-2 spike protein of SEQ ID NO. 20; which nucleic acid sequence if of SEQ ID NO. 21.

Based on the above nucleic acid sequences, the skilled artisan is fully taught with the nucleic acid sequence of any of the HR1-derived polypeptide of the present disclosure.

The present disclosure also pertains to any expression vector comprising a nucleic acid encoding any of the HR1-derived polypeptides described herein.

The present disclosure also concerns any recombinant host cell comprising a nucleic acid encoding any of the HR1-derived polypeptides described herein or an expression vector comprising the said nucleic acid, which expression vector allows expression of the said HR1-derived polypeptide, when comprised in an appropriate host cell..

The disclosure further relates to recombinant DNA constructs comprising the nucleic acids of the disclosure or variants, homologues or derivatives thereof. The constructs of the disclosure may further comprise additional elements such as promoters, regulatory and control elements, translation, expression and other signals, operably linked to a nucleic acid encoding a HR1-derived polypeptide of the disclosure.

Expression vectors are typically self-replicating DNA or RNA constructs containing the desired nucleic acid, and operably linked genetic control elements that are recognized in a suitable host cell and effect expression of the desired nucleic acids. These control elements are capable of effecting expression within a suitable host. Generally, the genetic control elements can include a prokaryotic promoter system or a eukaryotic promoter expression control system. This typically includes a transcriptional promoter, an optional operator to control the onset of transcription, transcription enhancers to elevate the level of RNA expression, a sequence that encodes a suitable ribosome binding site, sequences that terminate transcription and translation and so forth. Expression vectors may contain an origin of replication that allows the vector to replicate independently of the host cell.

Accordingly, the term, control and regulatory elements includes promoters, terminators and other expression control elements. For instance, any of a wide variety of expression control sequences that control the expression of a DNA sequence when operatively linked to it may be used in these vectors to express DNA sequences encoding any desired polypeptide of this disclosure. A vector may additionally include appropriate restriction sites, antibiotic resistance markers or other markers for selection of vector-containing cells. Plasmids are the most commonly used form, of vector but other forms of vectors which serve an equivalent function and which are, or become, known in the art are suitable for use herein.

### Immunogenic compositions

The present disclosure further relates to an immunogenic composition comprising one or more HR1-derived polypeptides described herein, or alternatively one or more expression vectors described herein, or alternatively one or more live vectors described herein, such as live recombinant bacteria or attenuated recombinant viruses allowing the expression of one or more HR1-derived polypeptides described herein.

Thus, the present disclosure also relates to an immunogenic composition comprising one or more HR1-derived polypeptide comprising 10 or more consecutive amino acids of the polypeptide of SEQ ID NO. 8, or a polypeptide derivative thereof. The said immunogenic compositions encompass an immunogenic composition comprising one or more HR1-derived polypeptide comprising 10 or more consecutive amino acids of a peptide selected in the group consisting of SEQ ID NO. 1 to SEQ ID NO. 5, or a polypeptide derivative thereof.

The present disclosure also further relates to an immunogenic composition comprising a nucleic acid encoding one or more HR1-derived polypeptides described herein, allowing the expression of these one or more polypeptides by cells of the subject that has been administered therewith.

Thus, the present disclosure also relates to an immunogenic composition comprising one or more nucleic acids encoding HR1-derived polypeptide comprising 10 or more consecutive amino acids of the polypeptide of SEQ ID NO. 8, such as the nucleic acids of SEQ ID NO. 11, 12, 13, 14,15, 16,17 and 18.

The term "immunogenic composition", as used herein, relates to a composition capable of eliciting an immunogenic reaction in mammals, preferably in mouse, rat and/or human, most preferably in human.

By "immunogenic composition" it is herein intended a substance which is able to induce an immune response in an individual, and for example to induce the production of antibodies directed against a HR1-derived polypeptide of the present disclosure.

Preferably, an immunogenic composition of the present disclosure, when administered to a subject in need thereof, raises an immune response that leads to preventing, lowering of blocking NKp44L membrane expression by cells of the said subject, most preferably either (i) a subject at risk of being infected with a SARS-COV-2 virus or (ii) a subject which has been infected with a SARS-COV-2 virus.

In some embodiments, because the said immune response comprises raising antibodies directed against a specific region of the SARS-COV-2 spike protein, an immunogenic composition of the present disclosure may also raise an immune response leading to a neutralization of the SARS-COV-2 virus particles present in the body of the infected subject and thus behave as a vaccine composition against a SARS-COV-2 virus.

Thus, the present disclosure also relates to a vaccine composition against a SARS-COV-2 virus, comprising a HR1-derived peptide as described herein, or a nucleic acid encoding a HR1-derived polypeptide as described herein. Otherwise said, any of the immunogenic compositions described in the present disclosure may be used as a vaccine composition against the infection of a subject with a SARS-COV-2 virus.

### Immunogenic compositions comprising HR1-derived polypeptides

A HR1-derived polypeptide as described herein can be produced by any known cloning technology or by any known method of chemical synthesis.

For example, DNA encoding a HR1-derived polypeptide as described herein may be prepared by use of a cloning technology and the be inserted into an autonomously replicable vector to prepare a recombinant DNA. The recombinant DNA is introduced into an appropriate host, such as *Escherichia coli, Bacillus subtilis, Actinomyces,* yeast, filamentous fungus, a plant cell, an insect cell and an animal cell, to obtain a transformant. From the cultured product of the transformant, a HR1-derived polypeptide can be obtained. Alternatively, DNA encoding a HR1-derived polypeptide is prepared and subjected to an acellular protein-synthesis system using wheat germ and a cell extract from *Escherichia coli,* to synthesize the desired HR1-derived polypeptide of the disclosure.

Moreover, using a customary chemical synthesis method for a HR1-derived polypeptide, such as a "solid phase method" or "a liquid phase method", amino acids are successively connected and extended by dehydration/condensation, according to peptide synthesis methods that are well known in the art.

In some preferred embodiments, a HR1-derived polypeptide of the present disclosure is present in an immunogenic composition under the form of an immunogenic product wherein the said HR1-derived polypeptide is linked to a carrier molecule.

In some embodiments, the said carrier molecule may be a carrier protein.

In some other embodiments, the said carrier molecule may be a synthetic polymer.

In some embodiments where the desired HR1-derived polypeptide is linked to a carrier protein, then an immunogenic product consisting of a fusion protein containing both a HR1-derived polypeptide and the desired carrier protein may be synthesized by a recombinant DNA technique.

In some other embodiments where the desired HR1-derived polypeptide is linked to a carrier molecule, an immunogenic product comprising the antigenic HR1-derived polypeptide can be synthesized by covalently binding a plurality of the said HR1-derived polypeptides of the disclosure to a given carrier molecule, such as to a given protein carrier molecule. In order to enhance peptide immunogenicity, a HR1-derived polypeptide of the present disclosure can be covalently linked ("conjugated") to another molecule, preferably to a larger molecule, which serves as a carrier, the resulting product consisting of an "immunoconjugate" according to the present disclosure.

Attachment of the peptide to the carrier molecule may be performed by one of several methods, including linking through a peptide Lys using glutaraldehyde (Reichlin, Methods Enzymol. 70: 159-165, 1980) or DCC procedures (for example, Atassi et al., Biochem. Biophys. Acta 670: 300-302, 1981), through a Peptide Asp or Glu using DCC (Bauminger et al., Methods Enzymol 70: 151-159, 1980), through a peptide Tyr using bis-diazotized benzidine (Walter et al., Proc. Nat. Acad. Sci. USA 77: 5197-5200, 1980), through photochemical attachment sites (Parker et al., Cold Spring Harbor Symposium-Modern Approaches to Vaccines, Ed. Chanock & Lerner, Cold Spring Harbor Press, New York, 1984), or through a peptide Cys (Liu et al., Biochem. 18: 690-697, 1979).

Polypeptide carrier conjugates can be separated from excess free polypeptide by dialysis or gel filtration. The level of loading of the polypeptide on the carrier can be determined either using a radioactive tracer to establish the loading level in a particular procedure, or by quantitative amino acid analysis of the conjugate, in comparison with the unloaded carrier. It is convenient, when using the latter technique, to incorporate a unique non-natural amino acid into the polypeptide, at the N-terminal or C-terminal side, such as Nle, which can then serve as a quantitative marker for polypeptide incorporation, as measured by amino acid analysis of the conjugate. This Nle can also function as a spacer between the antigenic site and any amino acid incorporated to facilitate attachment, such as Cys, Lys, or Tyr, as described above.

The carrier molecule to which the polypeptide is optionally bound can be selected from a wide variety of known carriers. Examples of carrier molecules for immunogenic purposes encompass proteins such as human or bovine serum albumin and keyhole limpet haemocyanin (KLH) and fatty acids. Other embodiments of carrier molecules to which an antigenic HR1-derived polypeptide may be covalently linked include bacterial toxins or toxoids, such as diphtheria, cholera, *E. coli* heat labile or tetanus toxoids, the *N. meninigitidis* outer membrane protein (European patent application n° EP0372501), synthetic peptides (European patent applications n° EP0378881 and n° EP0427347), heat shock proteins (PCT application n° WO93/17712), *Pertussis* proteins (PCT application n° WO98/58668), protein D from *H. influenzae* (PCT application n° WO00/56360) and toxin A or B from *C. difficile* (International patent application WO00/61761).

In an immunogenic composition wherein the HR1-derived polypeptide is linked to a carrier molecule consisting of a synthetic polymer, said synthetic polymer can be a multiple branch peptide construction comprising a core matrix comprised of lysine residues.

Radially branched systems using lysine skeletons in polymers have been used by J. P. Tam [Proc. Natl. Acad. Sci. U.S.A., 85, 5409-5413 (1988)] to develop antigens without the use of carriers. Those antigens were designed to generate vaccines against a variety of diseases.

The core matrix is preferably a dendritic polymer which is branched in nature, preferably with each of the branches thereof being identical. The core matrix is based on a core molecule which has at least two functional groups to which molecular branches having terminal functional groups are covalently bonded. Exemplary for use to form the core matrix is lysine. A central lysine residue is bonded to two lysine residues, each through its carboxyl group, to one of the amino groups of the central lysine residue. This provides a molecule with four amino groups, which may be a core matrix for a structure comprising four molecules of the selected HR1-desired polypeptide. The manufacture of the above structures has been known in the art. See, e.g., Tam et al., J. Immun. 148, 914-920 (1992) and Wang et al., Science, 254, 285-288 (1991).

Additionally, spacers between said HR1-derived polypeptide and said carrier protein or synthetic polymer can be added. A peptide linker sequence may be employed to separate the first and second HR1-derived polypeptide molecules by a distance sufficient to ensure that each HR1-derived polypeptide molecule folds into its secondary and tertiary structures. Such a peptide linker sequence is incorporated into the fusion protein using standard techniques well known in the art. Suitable peptide linker sequences may be chosen based on the following factors: (1) their ability to adopt a flexible extended conformation; (2) their inability to adopt a secondary structure that could interact with functional epitopes on the first and second polypeptides; and (3) the lack of hydrophobic or charged residues that might react with the polypeptide functional epitopes. Preferred peptide linker sequences contain Gly, Asn and Ser residues. Other near neutral amino acids, such as Thr and Ala may also be used in the linker sequence. Amino acid sequences which may be usefully employed as linkers include those disclosed in Maratea et al., Gene 40:39-46, 1985; Murphy et al., Proc. Natl. Acad. Sci. USA 83:8258-8262, 1986; U.S. Pat. No. 4,935,233 and U.S. Pat. No. 4,751,180. The linker sequence may generally be from 1 to about 50 amino acids in length. Linker sequences are not required when the first and second polypeptides have non-essential N-terminal amino acid regions that can be used to separate the functional domains and prevent steric interference.

Preferably, said carrier protein is selected from the group consisting of keyhole limpet hemocyanin (KLH), bovine serum albumin, diphtheria toxoid and CRM197, which are all protein carrier molecules well known in the art.

Incidentally, the CRM197 protein consists of a non-toxic mutant of the well-known diphtheria toxin, which mutant was initially described by Uchida et al. (1973, J. Biol. Chem., Vol. 248 : 3838-3844). The CRM197 mutant protein was initially described as the translation product of the mutanttox97gene where a G→A transition led to the substitution of the glycine (G) residue at position 52 of the wild-type diphtheria toxin with a glutamic acid residue (E).

### Immunogenic compositions comprising recombinant vectors

In some embodiments, an immunogenic composition of the present disclosure comprises an immunogenic vector expressing one or more of the HR1-derived polypeptides as described herein, such as recombinant a vaccinia virus expressing one or more of the HR1-derived polypeptides as described herein or a non-replicative vector such as a recombinant adenovirus vector comprising a nucleic acid encoding a HR1-derived polypeptide as described herein.

In some of these embodiments, the vaccinia virus may be a modified vaccinia virus Ankara (MVA).

Modified vaccinia virus Ankara (MVA) is a highly attenuated strain of vaccinia virus that was created by passaging vaccinia virus several hundred times in chicken embryo fibroblasts (CEF), thereby achieving growth selection. MVA shows a characteristic replication defect in mammalian cells (Mayr and Munz 1964; Meyer et al. 1991 ; Sutter and Moss 1992) and, thus, serves as one of the most advanced recombinant poxvirus vectors in preclinical research and human clinical trials for developing new vaccines against infectious disease and cancer (Gilbert 2013; Kremer ef a/. 2012; Volz and Sutter 2013).

Means and methods for introducing a nucleic acid molecule in accordance with the disclosure into a MVA are well known in the art and have been described e.g. in Drexler et al. 2000; Staib et al. 2004; or Kremer et al. 2012.

Preferably, the nucleic acid molecule encoding a HR1-derived polypeptide of the present disclosure is introduced into the MVA genome at an intergenic site and under transcriptional control of the vaccinia virus early/late promoter PmH5.

Another replicative vector that lay be used in an immunogenic composition of the present disclosure may consist of a measles-derived vector, such as reviewed for example by Phanramphoei et al. (2018, Microbes Infect, Vol. 20(9) : 493-500).

Non-replicative vectors may also be used, such as adenovirus vectors, tha are well known by the skilled artisan. The skilled artisan may for instance refer to the review by Tatsis et al. (2004, Molecular Therapy, Vol. 10(4) : 616). Are encompassed herein human adenoviruses of any of the serotypes A, B, C, D, E and F. Adenoviruses of serotype A encompass Ad-12, Ad-18 and Ad-31. Adenoviruses of serotype B1 encompass Ad-3, Ad-7, Ad-16, Ad-21 and Ad-50. Adenoviruses of serotype B2 encompass Ad-11, Ad-14, Ad-34 and Ad-35. Adenoviruses of serotype C encompass Ad-1, Ad-2, Ad-5 and Ad-6. Adenoviruses of serotype D encompass Ad-8, Ad-9, Ad-10, Ad-13, Ad-15, Ad-17, Ad-19, Ad-20, Ad-22, Ad-23, Ad-24, Ad-25, Ad-26, Ad-27, Ad-28, Ad-29, Ad-30, Ad-32, Ad-33, Ad-36, Ad-37, Ad-38, Ad-39, Ad-42, Ad-43, Ad-44, Ad-45, Ad-46, Ad-47, Ad-48, Ad-49 and Ad-51. Adenoviruses of serotype E encompass Ad-4. Adenoviruses of serotype F encompass Ad-40 and Ad-41.

### Immunogenic compositions comprising nucleic acids.

The present disclosure further pertains to an immunogenic composition comprising a nucleic acid that induces the expression of one or more HR1-derived polypeptides as described herein, when suitably administered to a subject in need thereof.

Most preferably, the said nucleic acid consists of a ribonucleic acid capable of inducing the expression of one or more HR1-derived polypeptides as described herein, when suitably administered to a subject in need thereof.

Thus, the present disclosure also concerns ribonucleic acid (RNA) immunogenic compositions that build on the knowledge that RNA, *e.g.*, messenger RNA (mRNA)) can safely direct the body's cellular machinery to produce nearly any protein of interest, from native proteins to antibodies and other entirely novel protein constructs that can have therapeutic activity inside and outside of cells. The RNA (e.g., mRNA) immunogenic compositions of the present disclosure may be used to prevent or block the induction of NKp44L membrane expression in subjects infected with a SARS-COV-2 virus.

The RNA (e.g., mRNA) immunogenic compositions have valuable properties in that they may produce high antibody titers and may produce responses earlier than polypeptide-containing immunogenic compositions. While not wishing to be bound by theory, it is believed that the RNA (*e.g*, mRNA) immunogenic compositions, as mRNA nucleic acids, are appropriately designed to produce the appropriate protein conformation upon translation as the RNA (*e.g.*, mRNA) immunogenic compositions co-opt natural cellular machinery. Further, RNA (*e.g.*, mRNA) immunogenic compositions may be presented to the cellular system in a more native fashion as compared to polypeptide-containing immunogenic compositions.

Provided herein, in some embodiments, is a ribonucleic acid (RNA) (*e.g.*, mRNA) immunogenic composition, comprising at least one (*e.g.*, at least 2, 3, 4 or 5) RNA (*e.g.*, mRNA) nucleic acid having an open reading frame encoding at least one (*e.g.*, at least 2, 3, 4 or 5) HR1-derived polypeptide of the present disclosure, or any combination of two or more of the foregoing HR1-derived polypeptides.

Also provided herein is a RNA (e.g., mRNA) immunogenic composition, which is formulated in a nanoparticle (*e.g.*, a lipid nanoparticle).

In some embodiments, the nanoparticle has a mean diameter of 50-200 nm. In some embodiments, the nanoparticle is a lipid nanoparticle. In some embodiments, the lipid nanoparticle comprises a cationic lipid, a PEG-modified lipid, a sterol and a non-cationic lipid. In some embodiments, the lipid nanoparticle comprises a molar ratio of about 20-60% cationic lipid, 0.5- 15% PEG-modified lipid, 25-55% sterol, and 25% non-cationic lipid. In some embodiments, the cationic lipid is an ionizable cationic lipid and the non-cationic lipid is a neutral lipid, and the sterol is a cholesterol. In some embodiments, the cationic lipid is selected from 2,2-dilinoleyl-4-dimethylaminoethyl-[1,3]-dioxolane (DLin-KC2-DMA), dilinoleyl-methyl-4-dimethylaminobutyrate (DLin-MC3-DMA), and di((Z)-non-2-en- 1-yl) 9-((4-(dimethylamino)butanoyl)oxy)heptadecanedioate (L319).

For a detailed description of immunogenic compositions comprising ribonucleic acids, the skilled artisan may refer to the PCT application, published under n° WO 2017/070626 to Modernatx, Inc.

### Adjuvants

In some aspects, the immunogenic composition of the present disclosure contains an adjuvant.

"Adjuvants" as used herein, can include aluminum hydroxide and aluminum phosphate, saponins e.g., Quil A, QS-21 (Cambridge Biotech Inc., Cambridge MA), GPI-0100 (Galenica Pharmaceuticals, Inc., Birmingham, AL), water-in-oil emulsion, oil-in-water emulsion, water-in-oil-in-water emulsion. The emulsion can be based in particular on light liquid paraffin oil (European Pharmacopea type); isoprenoid oil such as squalane or squalene; oil resulting from the oligomerization of alkenes, in particular of isobutene or decene; esters of acids or of alcohols containing a linear alkyl group, more particularly plant oils, ethyl oleate, propylene glycol di-(caprylate/caprate), glyceryl tri-(caprylate/caprate) or propylene glycol dioleate; esters of branched fatty acids or alcohols, in particular isostearic acid esters. The oil is used in combination with emulsifiers to form the emulsion. The emulsifiers are preferably nonionic surfactants, in particular esters of sorbitan, of mannide (e.g. anhydromannitol oleate), of glycol, of polyglycerol, of propylene glycol and of oleic, isostearic, ricinoleic or hydroxystearic acid, which are optionally ethoxylated, and polyoxypropylene -polyoxyethylene copolymer blocks, in particular the Pluronic products, especially L121. See Hunter et al., The Theory and Practical Application of Adjuvants (Ed.Stewart-Tull, D. E. S.), JohnWiley and Sons, NY, pp51-94 (1995) and Todd et al., Vaccine 15:564-570 (1997). Exemplary adjuvants are the SPT emulsion described on page 147 of"Vaccine Design, The Subunit and Adjuvant Approach" edited by M. Powell and M. Newman, Plenum Press, 1995, and the emulsion MF59 described on page 183 of this same book.

A further instance of an adjuvant is a compound chosen from the polymers of acrylic or methacrylic acid and the copolymers of maleic anhydride and alkenyl derivative. Advantageous adjuvant compounds are the polymers of acrylic or methacrylic acid which are cross-linked, especially with polyalkenyl ethers of sugars or polyalcohols. These compounds are known by the term carbomer (Phameuropa Vol. 8, No. 2, June 1996). Persons skilled in the art can also refer to U.S. Patent No. 2,909,462 which describes such acrylic polymers cross- linked with a polyhydroxylated compound having at least 3 hydroxyl groups, preferably not more than 8, the hydrogen atoms of at least three hydroxyls being replaced by unsaturated aliphatic radicals having at least 2 carbon atoms. The preferred radicals are those containing from 2 to 4 carbon atoms, e.g. vinyls, allyls and other ethylenically unsaturated groups. The unsaturated radicals may themselves contain other substituents, such as methyl. The products sold under the name Carbopol; (BF Goodrich, Ohio, USA) are particularly appropriate. They are cross-linked with an allyl sucrose or with allyl pentaerythritol. Among then, there may be mentioned Carbopol 974P, 934P and 97 IP. Most preferred is the use of Carbopol 97 IP. Among the copolymers of maleic anhydride and alkenyl derivative, are the copolymers EMA (Monsanto), which are copolymers of maleic anhydride and ethylene. The dissolution of these polymers in water leads to an acid solution that will be neutralized, preferably to physiological pH, in order to give the adjuvant solution into which the immunogenic, immunological or vaccine composition itself will be incorporated.

Further suitable adjuvants include, but are not limited to, the RIBI adjuvant system (Ribi Inc.), Block co-polymer (CytRx, Atlanta GA), SAF-M (Chiron, Emeryville CA), monophosphoryl lipid A, Avridine lipid-amine adjuvant, heat-labile enterotoxin from E. coli (recombinant or otherwise), cholera toxin, IMS 1314 or muramyl dipeptide, or naturally occurring or recombinant cytokines or analogs thereof or stimulants of endogenous cytokine release, among many others.

Immuno-adjuvant agents encompass, but are not limited to, Stimulon^{™}, QS-21 (Aquila Biopharmaceuticals, Inc., Framingham, Mass.); MPL^{™} (3-O-deacylated monophosphoryl lipid A; Corixa, Hamilton, Mont.), 529 (an amino alkyl glucosamine phosphate compound, Corixa, Hamilton, Mont.), IL-12 (Genetics Institute, Cambridge, Mass.); GM-CSF (Immunex Corp., Seattle, Wash.); N-acetyl-muramyl-L-theronyl-D-isoglutamine (thr-MDP); N-acetyl-nor-muramyl-L-alanyl-D-isoglutamine (CGP 11637, referred to as nor-MDP); N-acetylmuramyl-L-alanyl-D-isoglutaminyl-L-alanine-2-(1'-2'-dipalmitoyl-sn-glycero-3-hydroxyphosphoryloxy-ethylamine) (CGP 19835A, referred to as MTP-PE); and cholera toxin. Other immuno-adjuvant agents or compounds which may be used encompass non-toxic derivatives of cholera toxin, including its A subunit, and/or conjugates or genetically engineered fusions of the N. meningitidis polypeptide with cholera toxin or its B subunit ("CTB"), procholeragenoid, fungal polysaccharides, including schizophyllan, muramyl dipeptide, muramyl dipeptide ("MDP") derivatives, phorbol esters, the heat labile toxin of E. coli, block polymers or saponins.

### Additional compounds

In a preferred embodiment, an immunogenic composition according to the disclosure further comprises one or more components selected from the group consisting of surfactants, absorption promoters, water absorbing polymers, substances which inhibit enzymatic degradation, alcohol, organic solvents, oils, pH controlling agents, preservatives, osmotic pressure controlling agents, propellants, water and mixture thereof.

Examples of appropriate supplementary carriers include, but are not limited to, sterile water, saline, buffers, phosphate-buffered saline, buffered sodium chloride, vegetable oils, Minimum Essential Medium (MEM), MEM with HEPES buffer, etc.

Examples of suitable secondary adjuvants include, but are not limited to, stabilizers; emulsifiers; aluminium hydroxide; aluminium phosphate; pH adjusters such as sodium hydroxide, hydrochloric acid, etc.; surfactants such as Tween^{®} 80 (polysorbate 80, commercially available from Sigma Chemical Co., St. Louis, Mo.); liposomes; iscom adjuvant; synthetic glycopeptides such as muramyl dipeptides; extenders such as dextran or dextran combinations, for example, with aluminium phosphate; carboxypolymethylene; bacterial cell walls such as mycobacterial cell wall extract; their derivatives such as Corynebacterium parvum; Propionibacterium acne; Mycobacterium bovis, for example, Bovine Calmette Guerin (BCG); vaccinia or animal poxvirus proteins; subviral particle adjuvants such as orbivirus; cholera toxin; N,N-dioctadecyl-N',N'-bis(2-hydroxyethyl)-propanediamine (avridine); monophosphoryl lipid A; dimethyldioctadecylammonium bromide (DDA, commercially available from Kodak, Rochester, N.Y.); synthetics and mixtures thereof. Desirably, aluminium hydroxide is admixed with other secondary adjuvants or an immunoadjuvant such as Quil A.

Examples of suitable stabilizers include, but are not limited to, sucrose, gelatin, peptone, digested protein extracts such as NZ-Amine or NZ-Amine AS. Examples of emulsifiers include, but are not limited to, mineral oil, vegetable oil, peanut oil and other standard, metabolizable, non-toxic oils useful for injectable or intranasal immunogenic compositions.

These adjuvants are identified herein as "secondary" merely to contrast with the above-described immunoadjuvant compounds.

Conventional preservatives can be added to the immunogenic composition in effective amounts ranging from about 0.0001% to about 0.1% by weight. Depending on the preservative employed in the formulation, amounts below or above this range may be useful. Typical preservatives include, for example, potassium sorbate, sodium metabisulfite, phenol, methyl paraben, propyl paraben, thimerosal, etc.

### Further features of an immunogenic composition

Parenteral administration that is contemplated for the administration of an immunogenic composition according to the disclosure includes subcutaneous injections, submucosal injections, intravenous injections, intramuscular injections, transcutaneous injections, and infusion, most preferably intramuscular injections. Injectable preparations (e.g., sterile injectable aqueous or oleaginous suspensions) can be formulated according to the known art using suitable excipients, such as vehicles, solvents, dispersing, wetting agents, emulsifying agents, and/or suspending agents. These typically include, for example, water, saline, dextrose, glycerol, ethanol, corn oil, cottonseed oil, peanut oil, sesame oil, benzyl alcohol, benzyl alcohol, 1 ,3-butanediol, Ringer's solution, isotonic sodium chloride solution, bland fixed oils (e.g., synthetic mono- or diglycerides), fatty acids (e.g., oleic acid), dimethyl acetamide, surfactants (e.g., ionic and non-ionic detergents), propylene glycol, and/or polyethylene glycols. Excipients also may include small amounts of other auxiliary substances, such as pH buffering agents.

It is contemplated that the immunogenic composition may be freeze -dried (or otherwise reduced in liquid volume) for storage, and then reconstituted in a liquid before or at the time of administration. Such reconstitution may be achieved using, for example, vaccine -grade water.

The compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration preferably for administration to subjects, especially human. Associated with such container(s) can be a notice in the form prescribed by a governmental agency regulating the manufacture, use or sale of pharmaceuticals or biological products, which notice reflects approval by the agency of manufacture, use or sale for administration.

### Method of treatment

Further, the present disclosure provides a method for immunizing a subject comprising administering to such subject an immunogenic composition as described herein.

The term "immunizing" relates to an active immunization by the administration of an immunogenic composition to a subject to be immunized, thereby causing an immunological response against the antigen included in such immunogenic composition.

Preferably, immunization results in lessening of the incidence of the SARS-COV-2 infection in a subject or in the reduction in the severity of clinical signs caused by or associated with the SARS-COV-2 infection.

In some embodiments, it may also be expected that the immune response, that will comprise the raising of antibodies directed to a HR1-derived polypeptide of the disclosure; *i.e.* antibodies directed to a specific region of a SARS-COV-2 virus of low or no variability and which is involved in the fusion of the virus with the target cells, will lead to a neutralization of the virus and thus may be used as a vaccine composition against an infection of a subject with SARS-COV-2.

Further, the immunization of a subject in need of an immunogenic composition as provided herewith, results in preventing deleterious physiological effects caused by an infection of the said subject with a SARS-COV-2 virus. Immunization is aimed at causing an effective, long- lasting, immunological-response against a HR-1-derived polypeptide as described herein.It is to be understood that immunization may not be effective in all subjects immunized. However, the term requires that a significant portion of SARS-COV-2-infected subjects are effectively immunized.

Further, the present disclosure provides a method of treating or preventing clinical signs caused by a SARS-COV-2 virus in a subject in need thereof, wherein the method comprises administering to the subject an effective amount of an immunogenic composition as described herein.

In some embodiments wherein an immunogenic composition as described herein is used as a vaccine composition, the present disclosure also relates to a method of preventing an infection with a SARS-COV-2 virus in a subject in need thereof, wherein the method comprises administering to the subject an effective amount of an immunogenic composition as described herein.

The term "treating or preventing" refers to the lessening of the incidence of the particular SARS-COV-2 infection in infected subjects or the reduction in the severity of clinical signs caused by or associated with a SARS-COV-2 infection. Thus, the term "treating or preventing" also refers to the reduction of the severity of clinical signs normally associated with or caused by a SARS-COV-2 infection in subjects which subjects have received an effective amount of the immunogenic composition as provided herein in comparison to a group of subjects which subjects have not received such immunogenic composition. In the embodiments wherein an immunogenic composition is used as a vaccine composition, the term "preventing" further encompasses preventing an infection of a subject with a SARS-COV-2 virus.

The "treating or preventing" generally involves the administration of an effective amount of the immunogenic composition of the present disclosure to a subject in need of or that could benefit from such a treatment/prophylaxis. The term "treatment" encompasses the administration of the effective amount of the immunogenic composition once the subject is already infected with a SARS-COV-2 virus and wherein such subject already shows some clinical signs caused by or associated with such SARS-COV-2 infection. The term "prophylaxis" refers to the administration of a subject prior to any infection of such subject with a SARS-COV-2 virus or at least where such subject does not show any clinical signs caused by or associated with the infection by a SARS-COV-2 virus. The terms "preventing" and "prophylaxis" thus encompass embodiments referring to the administration of a subject prior to any infection with the aim of preventing an infection of the said subject with a SARS-COV-2 virus. The terms "prophylaxis" and "preventing" are used interchangeably in this description.

The term "an effective amount" as used herein means, but is not limited to an amount of antigen, that elicits or is able to elicit an immune response in a subject. Such effective amount is able to lessen the incidence of a SARS-COV-2 infection in a subject or to reduce the severity of clinical signs of a SARS-COV-2 infection.

The term "in need" or "of need", as used herein means that the administration/treatment is associated with the boosting or improvement in health or clinical signs or any other positive medicinal effect on health of the subjects which receive the immunogenic composition in accordance with the present disclosure.

In one specific aspect of the method or use according to the present disclosure said immunogenic composition is administered subcutaneously, intramuscularly, oral, via spray, via drinking water or by eye drop.

Suitable routes of administration conventionally used are oral or parenteral administration, such as intranasal, intravenous, intradermal, transdermal, intramuscular, intraperitoneal, subcutaneous, as well as inhalation, via spray, via drinking water or by eye drop. However, depending on the nature and contemplated mode of action, the immunogenic composition may be administered by other routes as well. For example, such other routes include intracutaneously, intravenously, intraperitoneally, intracutaneously or intrapulmonarily.

The immunogenic composition is, preferably, administered systemically, and most preferably intramuscularly.

The amount of an immunogenic compound to be administered to an individual is easily determined or adapted by the one skilled in the art, who is primarily guided by the effective amount range of a HR1-derived polypeptide, which includes the effective amount range of a HR1-derived polypeptide, *e.g.* selected from the group consisting of SEQ ID N° 1-5 and 8, and then by the molecular mass of the immunogenic compound he intends to administer.

In some embodiments, the amount of immunogenic polypeptide to be administered may be of about 1 µg to 1000 µg of HR1-derived polypeptide(s) contained thereinper dosage unit.

The amount of a HR1-derived polypeptide specified above also apply when the said HR1-derived polypeptide or a HR1-derived peptide is conjugated to a carrier molecule, in an immunogenic composition according to the disclosure.

In certain embodiments, an immunogenic composition is administered at least twice to an individual in need thereof. In these embodiments, the second step of administration of an immunogenic composition according to the disclosure is performed in a time period of from 2 weeks to 6 months after the first administration step.

In certain embodiments, an immunogenic composition is administered at least three times to an individual in need thereof. In these embodiments, the second step of administration of the said immunogenic composition according to the disclosure is performed in a time period of from 2 weeks to 6 months after the first administration step. In these embodiments, the third step of administration of the said immunogenic composition is performed in a time period of from more than 6 months to about one year after the first administration step.

In some embodiments, the said immunogenic composition is then again administered to an immunized individual, for example every 5 year-time period or every 10 year-time period.

Thus, the present disclosure also relates to an immunogenic composition as described herein, for its use for treating an infection of a subject with a SARS-COV-2 virus.

It relates to the use of a HR1-derived polypeptide of the disclosure, possibly linked to a carrier molecule, for preparing an immunogenic composition as described herein.

It also relates to a method of treating a subject infected with a SARS-COV-2 virus comprising as step of administering to the said subject an immunogenic composition as described herein.

The present disclosure also concerns an immunogenic composition as described herein for its use for reducing the deleterious effects of an infection of a subject by a SARS-COV-2 virus in a subject.

The present disclosure further relates to a vaccine composition comprising a HR1-derived polypeptide as described herein, which includes a vaccine composition comprising an immunogenic compound containing such a HR1-derived polypeptide, for its use for preventing an infection of a subject with a SARS-COV-2 virus.

It also pertains to the use of a HR1-derived polypeptide as described herein, which includes an immunogenic compound containing such a HR1-derived polypeptide, for its use for preventing an infection of a subject with a SARS-COV-2 virus for preparing a vaccine composition against a SARS-COV-2 virus.

This disclosure also concerns a method of preventing an infection with a SARS-COV-2 virus in a subject comprising as step of administering to the said subject a vaccine composition as described herein.

### Antibodies

The present disclosure also relates to an antibody directed against a HR1-derived polypeptide as described herein.

Thus, the present disclosure relates to an antibody directed against a HR1-derived polypeptide comprising 10 or more consecutive amino acids of the polypeptide of SEQ ID NO. 8, or of a polypeptide derivative thereof. This encompasses antibodies directed against a HR1-derived polypeptide comprising 10 or more consecutive amino acids of a peptide selected in the group consisting of SEQ ID NO. 1 to SEQ ID NO. 5, or a polypeptide derivative thereof.

Thus, the present disclosure encompasses antibodies that are obtained by immunizing a human or an animal individual, preferably a non-human mammal, with an immunogenic composition as described herein.

In some embodiments, the disclosure pertains to polyclonal antibodies obtained by immunizing a human or an animal individual with an immunogenic composition as described herein.

In some other embodiments, the disclosure pertains to monoclonal antibodies directed against a HR1-derived polypeptide as described herein, as well as to an antigen-binding fragment thereof.

The term "antibody" as used herein encompasses the whole antibodies as well as any antigen binding fragment (*i.e*.,"antigen-binding portion") or single chain thereof. An" antibody "refers to a glycoprotein comprising at least two heavy (H) chains and two light (L) chains inter-connected by disulfide bonds, or an antigen binding portion thereof. Each heavy chain is comprised of a heavy chain variable region (abbreviated herein as VH) and a heavy chain constant region (abbreviated herein as CH). Each light chain is comprised of a light chain variable region (abbreviated herein as VL) and a light chain constant region (abbreviated herein as CL). The VH and VL regions can be further subdivided into regions of hypervariability, termed "complementarity determining regions" (CDRs), interspersed with regions that are more conserved, termed "framework regions" (FRs). Each VH and VL is composed of three CDRs and four FRs, arranged from amino-termmus to carboxy-terminus in the following order: FRL CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (*e.g.*, effector cells) and the first component (C1q) of the classical complement system.

More specifically, an "antibody fragment" is a portion of an antibody such as F(ab')2, F(ab)2, Fab', Fab, and the like. Regardless of structure, an antibody fragment binds with the same antigen that is recognized by the intact antibody. For example, an anti-(HR1-derived polypeptide according to the present disclosure) monoclonal antibody fragment binds an epitope of a HR1-derived polypeptide according to the present disclosure, *e.g.* a HR1-drived polypeptide selected in the group consisting of the polypeptides of SEQ ID NO. 1 to 5 and 8. The term "antibody fragment" also includes a synthetic or a genetically engineered polypeptide that binds to a specific antigen, such as polypeptides consisting of the light chain variable region,"Fv" fragments consisting of the variable regions of the heavy and light chains, recombinant single chain polypeptide molecules in which light and heavy variable regions are connected by a peptide linker "scFv proteins"), and minimal recognition units consisting of the amino acid residues that mimic the hypervariable region.

Methods for preparing antibodies are known to the art (See, for example, Harlow & Lane (1988) Antibodies: a Laboratory Manual, Cold Spring Harbor Lab., Cold Spring Harbor, NY). Monoclonal antibodies may be prepared from a single B cell line taken from the spleen or lymph nodes of immunized animals, in particular rats or mice, by fusion with immortalized B cells under conditions which favor the growth of hybrid cells. The technique of generating monoclonal antibodies is described in many articles and textbooks. Spleen or lymph node cells of these animals may be used in the same way as spleen or lymph node cells of protein- immunized animals, for the generation of monoclonal antibodies as described in the art. The techniques used in generating monoclonal antibodies are further described in by Kohler and Miistein (Nature 256; 495-497, (1975), and in USP 4,376,110). Antibodies that are isolated from organisms other than humans, such as mice, rats, rabbits, cows, can be made more human-like through chimerization or humanization, according to methods well known in the art.

Suitable monoclonal antibodies which are reactive as described herein may be prepared by known techniques, for example those disclosed in "Monoclonal Antibodies; A manual of techniques", H Zola (CRC Press, 1988) and in "Monoclonal Hybridoma Antibodies: Techniques and Application", S G R Hurrell (CRC Press, 1982).

A further embodiment encompasses humanized antibodies where the regions of the murine antibody that contacted the antigen, the Complementarity Determining Regions (CDRs) were transferred to a human antibody framework. Such antibodies are almost completely human and seldom cause any harmful antibody responses when administered to patients. Several chimeric or humanized antibodies have been registered as therapeutic drugs and are now widely used within various indications (Borrebaeck & Carlsson, 2001, Curr. Opin. Pharmacol. 1: 404-408). Suitably prepared non-human antibodies can be "humanized" in known ways, for example by inserting the CDR regions of mouse antibodies into the framework of human antibodies. Humanized antibodies can be made using the techniques and approaches described in Verhoeyen et al (1988) Science, 239, 1534-1536, and in Kettleborough et al, (1991) Protein Engineering, 14 (7), 773-783.

The present disclosure relates to an antibody directed against a HR1-derived polypeptide as described herein for its use as a medicament/

It concerns the use of an antibody directed against a HR1-derived polypeptide as described herein for preparing a medicament.

This disclosure also concerns a method for treating a subject in need thereof comprising a step of administering an antibody directed against a HR1-derived polypeptide as described herein to the said subject.

The present disclosure further relates to an antibody directed against a HR1-derived polypeptide as described herein for treating a subject infected with a SARS-COV-2 virus.

This disclosure also pertains to the use of an antibody directed against a HR1-derived polypeptide as described herein for preparing a medicament for treating a subject infected a SARS-COV-2 virus.

This disclosure also concerns a method for treating a subject infected with a SARS-COV-2 virus comprising a step of administering an antibody directed against a HR1-derived polypeptide as described herein to the said subject.

The present disclosure further relates to an antibody directed against a HR1-derived polypeptide as described herein for reducing the clinical symptoms (signs) of a disease associated with an infection of a subject with a SARS-COV-2 virus.

This disclosure also concerns the use of to an antibody directed against a HR1-derived polypeptide as described herein for preparing a medicament for reducing the clinical symptoms (signs) of a disease associated with an infection of a subject with a SARS-COV-2 virus.

This disclosure also pertains to a method for reducing the clinical symptoms (signs) of a disease associated with an infection of a subject with a SARS-COV-2 virus comprising a step of administering an antibody directed against a HR1-derived polypeptide as described herein to the said subject.

The present disclosure further relates to an antibody directed against a HR1-derived polypeptide as described herein for preventing an infection of a subject with a SARS-COV-2 virus.

This disclosure also concerns the use of to an antibody directed against a HR1-derived polypeptide as described herein for preparing a medicament for preventing an infection of a subject with a SARS-COV-2 virus.

This disclosure also pertains to a method preventing an infection of a subject with a SARS-COV-2 virus comprising a step of administering an antibody directed against a HR1-derived polypeptide as described herein to the said subject.

### Diagnosis methods

Another aspect of the present disclosure concerns the detection of antibodies directed against a HR1-derived polypeptide as described herein, in particular the detection of such antibodies when present in a body fluid of a subject.

According to this other aspect of the present disclosure, detecting antibodies directed against a HR1-derived polypeptide in a body fluid sample of a subject allows (i) determining the presence of a SARS-COV-2 virus in the said body fluid sample and also (ii) determining the probability of the said subject to experiment deleterious effects caused by the induction of NKp44L membrane expression.

According to this other aspect, detecting and/or quantifying antibodies directed against a HR1-derived polypeptide in a body fluid sample of a subject allows monitoring the efficiency of the administration of the said subject with an immunogenic composition disclosed in the present description.

Without wishing to be bound by any particular theory, the inventors believe that the presence and/or the level of antibodies directed against a HR1-derived polypeptide or against a HR1-derived peptide as described herein consists of a biological marker of physiological dysfunctions caused by a SARS-COV-2 infection.

Accordingly, the disclosure also concerns a method for the *in vitro* assessment of the infection of an individual with a SARS-COV-2 virus, wherein said method comprises the step of detecting in a sample from said individual, antibodies directed against a HR1-derived polypeptide as described herein.

The sole detection of antibodies directed against a HR1-derived polypeptide as described herein might not be sufficient for a global accurate clinical diagnosis, or prognosis, or determination of the progression status of the disease within the patient tested. Thus, the detection of antibodies directed against a HR1-derived polypeptide as described herein might be completed by, or combined with, other diagnosis or prognosis markers known in the art.

The detection of antibodies directed against a HR1-derived polypeptide as described herein can be achieved using known techniques such as ELISA or RIA tests.

This disclosure relates to an *in vitro* method for detecting and/or quantifying antibodies directed against a HR1-derived polypeptide as described herein in a sample, comprising the following steps:
a) bringing into contact a sample to be tested with one or more HR1-derived polypeptide as described herein, and
b) detecting and/or quantifying the formation of complexes between the said HR1-derived polypeptide and antibodies present in the said sample.

The present disclosure also concerns a kit for detecting and/or quantifying antibodies directed HR1-derived polypeptide or HR1-derived peptide in a sample, comprising:
a) one or more HR1-derived polypeptide as described herein, and
b) one or more reagents for detecting complexes formed between the said HR1-derived polypeptide and antibodies present in the said sample.

In some embodiments, the sample to be tested consists of a sample previously collected form an individual, which includes (i) an individual who is suspected to be infected by SARS-COV-2 virus and (ii) an individual who has been infected by a SARS-COV-2 virus.

In some embodiments, the said sample consists of a preparation expected to contain antibodies directed against HR1-derived polypeptide, such as (i) a preparation of antibodies purified from samples collected from a mammal immunized with an immunogenic composition of the present disclosure and (ii) a preparation of monoclonal antibodies or of recombinant antibodies directed against HR1-derived polypeptide.

In some embodiments of the detection method above, the HR1-derived polypeptide(s) is (are) immobilized on a substrate.

In some embodiments, HR1-derived polypeptide may be used as a reagent for diagnosing the infection of an individual with a SARS-COV-2 virus.

Thus, the present disclosure also concerns a method for the diagnosis of an infection with a SARS-COV-2 virus in an individual, comprising the following steps:
a) bringing into contact a sample from the said individual with one or more HR1-derived polypeptides or HR1-derived peptides, and
b) detecting formation of complexes between the said HR1-derived polypeptides and antibodies present in the said sample.

In some embodiments of step b) of the method, the complexes between the said HR1-derived polypeptides and antibodies, when present, are quantified.

This disclosure also relates to a kit for the diagnosis of an infection with a SARS-COV-2 virus in an individual, comprising:
a) one or more HR1-derived polypeptides as described herein, and
b) one or more reagents for detecting complexes formed between the said HR1-derived polypeptides and antibodies present in a sample collected from said individual.

In some embodiments of the diagnosis method or of the diagnosis kit above, the said HR1-derived polypeptide(s) is (are) immobilized on a substrate, as it is disclosed further in the present specification.

According to the method above, a SARS-COV-2 infection is determined if the formation of complexes between the HR1-derived polypeptide(s) and antibodies contained in the sample previously collected from the tested individual are detected.

According to the diagnosis method above, the level of the immune response of a SARS-COV-2-infected individual against the said virus is determined by quantifying the complexes formed between the HR1-derived polypeptide(s) and antibodies contained in the sample previously collected from the tested individual.

In some embodiments, a HR1-derived polypeptide may be used as a reagent for performing a prognosis of progression of the infection of an individual with SARS-COV-2 virus.

Thus, the present disclosure also concerns a method for a prognosis of progression of an infection with a SARS-COV-2 virus in an individual, comprising the following steps:
a) bringing into contact a sample from the said individual with one or more HR1-derived polypeptide as described herein, and
b) detecting and quantifying the formation of complexes between the said HR1-derived polypeptide and antibodies present in the said sample.

This disclosure also relates to a kit for the prognosis of progression of an infection with a SARS-COV-2 virus in an individual, comprising:
a) one or more HR1-derived polypeptide as described herein, and
b) one or more reagents for detecting complexes formed between the said HR1-derived polypeptide and antibodies present in a sample collected from said individual.

According to the method above, a SARS-COV-2 infection is determined if the formation of complexes between the HR1-derived polypeptide and antibodies contained in the sample previously collected from the tested individual are detected.

According to the prognosis method above, the level of the immune response of a SARS-COV-2-infected individual against the said virus is determined at step b).

When performing the prognosis method above, deleterious physiological effects are expected when a high level of antibodies directed to HR1-derived polypeptide is measured at step b). Conversely, a more favourable outcome is expected when a low level of antibodies directed to HR1-derived polypeptide is measured at step b).

As used herein, a "high" level of antibodies directed against HR1-derived polypeptide is determined by comparison with one or more reference values.

The diagnosis method above, the prognosis method above, as well as the kits for performing the said methods, may be used for monitoring the efficiency of an anti-SARS-COV-2 medical treatment in a SARS-COV-2-infected individual.

This disclosure also relates to a method for monitoring a medical treatment against a SARS-COV-2-induced disease in a SARS-COV-2-infected individual, comprising the steps of:
a) administering a medical treatment against a SARS-COV-2-induced disease to the said SARS-COV-2-infected individual, and
b) determining the level of antibodies directed against a SARS-COV-2-induced disease in a sample collected from the said patient.

In some embodiments of the monitoring method, level of antibodies directed against a SARS-COV-2-induced disease is determined prior to administering a medical treatment against a SARS-COV-2-induced disease to the said individual, thus prior to step a) of the method.

In some embodiments of the monitoring method, especially when the medical treatment against a SARS-COV-2-induced disease comprises a plurality of steps of administering an appropriate pharmaceutical composition to the said individual, the monitoring method above is performed at each administration step of the said pharmaceutical composition, or only at one or more administration steps of the said pharmaceutical composition or at after the last administration step of the said pharmaceutical composition.

It is herein specified that embodiments of the anti-SARS-COV-2 medical treatment that may monitored according to the method above encompass immunization of the SARS-COV-2-infected individual with an immunogenic composition comprising a HR1-derived polypeptide as described herein.

Phrases such as "sample containing an antibody" or "detecting an antibody in a sample" are not meant to exclude samples or determinations (detection attempts) where no antibody is contained or detected. In a general sense, this disclosure involves assays to determine whether an antibody produced in response to infection and during the course of the disease with a SARS-COV-2 virus is present in a sample, irrespective of whether or not it is detected.

Conditions for reacting peptides and antibodies so that they react specifically are well-known to those of skill in the art. See, e.g., Current Protocols in Immunology (Coligan et al., editors, John Wiley & Sons, Inc) or the Examples herein.

The diagnostic method comprises taking a sample of body fluid or tissue likely to contain antibodies. The antibodies can be, *e.g.,* of IgG, IgE, IgD, IgM, or IgA type.

Generally, IgM and/or IgA antibodies are detected, *e.g.* for the detection of early infection. IgG antibodies can be detected when some of the additional peptides discussed above are used in the method (*e.g.* peptides for the detection of flagellum proteins). The sample is preferably easy to obtain and may be serum or plasma derived from a venous blood sample or even from a finger prick. Tissue from other body parts or other bodily fluids, such as cerebro-spinal fluid (CSF), saliva, gastric secretions, mucus, etc. are known to contain antibodies and may be used as a source of the sample.

Once the HR1-derived polypeptide antigen and sample antibody are permitted to react in a suitable medium, an assay is performed to determine the presence or absence of an antibody-peptide reaction. Among the many types of suitable assays, which will be evident to a skilled worker, are immunoprecipitation and agglutination assays.

In embodiments of the disclosure, the assay may comprise (1) immobilizing the antibody(s) in the sample, adding a HR1-derived polypeptide of the disclosure, and then detecting the degree of antibody bound to the HR1-derived polypeptide, *e.g.* by the HR1-derived polypeptide being labelled or by adding a labelled substance (conjugate, binding partner), such as a labelled antibody, which specifically recognizes the HR1-derived polypeptide; (2) immobilizing a HR1-derived polypeptide of the disclosure, adding the sample containing an antibody(s), and then detecting the amount of antibody bound to the HR1-derived polypeptide, *e.g.* by adding a labelled substance (conjugate, binding partner), such as a labelled antibody, which specifically recognizes the antibody; or (3) reacting the HR1-derived polypeptide and the sample containing antibody(s) without any of the reactants being immobilized, and then detecting the amount of complexes of antibody and HR1-derived polypeptide, *e.g.* by the HR1-derived polypeptide being labelled or by adding a labelled substance (conjugate, binding partner), such as a labelled antibody, which specifically recognizes the peptide.

Immobilization of a HR1-derived polypeptide of the disclosure can be either covalent or non-covalent, and the non-covalent immobilization can be non-specific (*e.g.* non-specific binding to a polystyrene surface in e.g. a microtiter well). Specific or semi-specific binding to a solid or semi-solid carrier, support or surface, can be achieved by the peptide having, associated with it, a moiety which enables its covalent or non-covalent binding to the solid or semi-solid carrier, support or surface. For example, the moiety can have affinity to a component attached to the carrier, support or surface. In this case, the moiety may be, e.g., a biotin or biotinyl group or an analogue thereof bound to an amino acid group of the peptide, such as 6-aminohexanoic acid, and the component is then avidin, streptavidin or an analogue thereof. An alternative is a situation in which the moiety has the amino acid sequence His-His-His-His-His-His (SEQ ID NO: 19) and the carrier comprises a Nitrilotriacetic Acid derivative (NTA) charged with Ni⁺⁺ ions. Among suitable carriers, supports or surface are, e.g., magnetic beads or latex of co-polymers such as styrene-divinyl benzene, hydroxylated styrene-divinyl benzene, polystyrene, carboxylated polystyrene, beads of carbon black, non-activated or polystyrene or polyvinyl chloride activated glass, epoxy-activated porous magnetic glass, gelatin or polysaccharide particles or other protein particles, red blood cells, mono- or polyclonal antibodies or Fab fragments of such antibodies.

The protocols for immunoassays using antigens for detection of specific antibodies are well known in art. For example, a conventional sandwich assay can be used, or a conventional competitive assay format can be used. For a discussion of some suitable types of assays, see Current Protocols in Immunology (*supra*). In a preferred assay, a peptide of the disclosure is immobilized to the solid or semi-solid surface or carrier by means of covalent or non-covalent binding, either prior to or after the addition of the sample containing antibody.

Devices for performing specific binding assays, especially immunoassays, are known and can be readily adapted for use in the present methods. Solid phase assays, in general, are easier to perform than heterogeneous assay methods which require a separation step, such as precipitation, centrifugation, filtration, chromatography, or magnetism, because separation of reagents is faster and simpler. Solid-phase assay devices include microtiter plates, flow-through assay devices, dipsticks and immunocapillary or immunochromatographic immunoassay devices.

In embodiments of the disclosure, the solid or semi-solid surface or carrier is the floor or wall in a microtiter well; a filter surface or membrane (e.g. a nitrocellulose membrane or a PVDF (polyvinylidene fluoride) membrane, such as an Immobilon membrane); a hollow fiber; a beaded chromatographic medium (e.g. an agarose or polyacrylamide gel); a magnetic bead; a fibrous cellulose matrix; an HPLC matrix; an FPLC matrix; a substance having molecules of such a size that the molecules with the peptide bound thereto, when dissolved or dispersed in a liquid phase, can be retained by means of a filter; a substance capable of forming micelles or participating in the formation of micelles allowing a liquid phase to be changed or exchanged without entraining the micelles; a watersoluble polymer; or any other suitable carrier, support or surface.

In some embodiments of the disclosure, the HR1-derived polypeptide is provided with a suitable label which enables detection. Conventional labels may be used which are capable, alone or in concert with other compositions or compounds, of providing a detectable signal. Suitable detection methods include, *e.g.*, detection of an agent which is tagged, directly or indirectly, with a fluorescent label by immunofluorescence microscopy, including confocal microscopy, or by flow cytometry (FACscan); detection of a radioactively labeled agent by autoradiography; electron microscopy; immunostaining; subcellular fractionation, or the like. In one embodiment, a radioactive element (*e.g.* a radioactive amino acid) is incorporated directly into a peptide chain; in another embodiment, a fluorescent label is associated with a HR1-derived polypeptide via biotin/avidin interaction, association with a fluorescein conjugated antibody, or the like. In one embodiment, a detectable specific binding partner for the antibody is added to the mixture. For example, the binding partner can be a detectable secondary antibody which binds to the first antibody. This secondary antibody can be labeled, *e.g.*, with a radioactive, enzymatic, fluorescent, luminescent, or other detectable label, such as an avidin/biotin system.

In embodiments of the disclosure, the detection procedure comprises visibly inspecting the antibody-peptide complex for a color change or inspecting the antibody-peptide complex for a physical-chemical change. Physical-chemical changes may occur with oxidation reactions or other chemical reactions. They may be detected by eye, using a spectrophotometer, or the like.

In one embodiment of the method, the HR1-derived polypeptide, or a mixture of HR1-derived polypeptides, is electro- or dot-blotted onto nitrocellulose paper. Subsequently, the biological fluid (*e.g.* serum or plasma) is incubated with the blotted antigen, and antibody in the biological fluid is allowed to bind to the antigen(s). The bound antibody can then be detected, *e.g.* by standard immunoenzymatic methods.

In another embodiment of the method, latex beads are conjugated to the antigen(s) of the disclosure. Subsequently, the biological fluid is incubated with the bead/peptide conjugate, thereby forming a reaction mixture. The reaction mixture is then analyzed to determine the presence of the antibodies.

One preferred assay for the screening of blood products or other physiological or biological fluids is an enzyme linked immunosorbent assay, i.e., an ELISA. Typically in an ELISA, the isolated HR1-derived polypeptide(s) of the disclosure is adsorbed to the surface of a microtiter well directly or through a capture matrix (i.e., antibody). Residual, non-specific protein-binding sites on the surface are then blocked with an appropriate agent, such as bovine serum albumin (BSA), heat-inactivated normal goat serum (NGS), or BLOTTO (a buffered solution of nonfat dry milk which also contains a preservative, salts, and an antifoaming agent). The well is then incubated with a biological sample suspected of containing anti-HR1-derived polypeptide antibodies. The sample can be applied neat, or more often it can be diluted, usually in a buffered solution which contains a small amount (0.1-5.0% by weight) of protein, such as BSA, NGS, or BLOTTO. After incubating for a sufficient length of time to allow specific binding to occur, the well is washed to remove unbound protein and then incubated with an optimal concentration of an appropriate anti-immunoglobulin antibody (e.g., for human subjects, an anti-human immunoglobulin from another animal, such as dog, mouse, cow, etc.) that is conjugated to an enzyme or other label by standard procedures and is dissolved in blocking buffer. The label can be chosen from a variety of enzymes, including horseradish peroxidase (HRP), beta-galactosidase, alkaline phosphatase, glucose oxidase, etc. Sufficient time is allowed for specific binding to occur again, then the well is washed again to remove unbound conjugate, and a suitable substrate for the enzyme is added. Color is allowed to develop and the optical density of the contents of the well is determined visually or instrumentally (measured at an appropriate wavelength). The cutoff OD value may be defined as the mean OD standard deviations (SDs) of at least 50 serum samples collected from individuals who are not infected by a SARS-COV-2 virus, or by other such conventional definitions. In the case of a very specific assay, OD+2 SD can be used as a cutoff value.

In one embodiment of an ELISA, a HR1-derived polypeptide of the disclosure is immobilized on a surface, such as a ninety-six-well ELISA plate or equivalent solid phase that is coated with streptavidin or an equivalent biotin-binding compound at an optimal concentration in an alkaline coating buffer and incubated at 4° C. overnight. After a suitable number of washes with standard washing buffers, an optimal concentration of a biotinylated form of a composition/antigen of this disclosure dissolved in a conventional blocking buffer is applied to each well; a sample is added; and the assay proceeds as above.

Another useful assay format is a lateral flow format. Antibody to human or animal antibody or staph A or G protein antibodies is labelled with a signal generator or reporter (*i.e.* colloidal gold) that is dried and placed on a glass fiber pad (sample application pad). The diagnostic HR1-derived polypeptide of the disclosure is immobilized on membrane, such as a PVDF (polyvinylidene fluoride) membrane (e.g. an Immobilon membrane (Millipore)) or a nitrocellulose membrane. When a solution of sample (blood, serum, etc) is applied to the sample application pad, it dissolves the colloidal gold labelled reporter and this binds to all antibodies in the sample. This mixture is transported into the next membrane (PVDF or nitrocellulose containing the diagnostic peptide) by capillary action. If antibodies against the HR1-derived polypeptide are present, they bind to the diagnostic peptide striped on the membrane generating a signal. An additional antibody specific to the colloidal gold labelled antibody (such as goat anti-mouse IgG) is used to produce a control signal.

It should be understood by the one of skill in the art that any number of conventional protein assay formats, particularly immunoassay formats, may be designed to utilize the isolated peptides of this disclosure for the detection of a SARS-COV-2 infection in a subject. This disclosure is thus not limited by the selection of the particular assay format, and is believed to encompass assay formats that are known to those of skill in the art.

Reagents for ELISA or other assays according to this disclosure can be provided in the form of kits. Such kits are useful for diagnosing infection with a SARS-COV-2 virus, using a sample from a subject (*e.g.* a human or other animal). Such a diagnostic kit can contain a HR1-derived polypeptide of the disclosure and, optionally, a system for (means enabling) detection of a HR1-derived polypeptide of the disclosure bound to an antibody directed against a HR1-derived polypeptide of the disclosure, and/or a surface to which the HR1-derived polypeptide can be bound. In one embodiment, a kit contains a mixture of suitable HR1-derived polypeptides or means for preparing such mixtures, and/or reagents for detecting peptide-antibody complexes.

The kit can include microtiter plates to which the HR1-derived polypeptide(s) of the disclosure have been pre-adsorbed, another appropriate assay device, various diluents and buffers, labelled conjugates or other agents for the detection of specifically bound antigens or antibodies, and other signal-generating reagents, such as enzyme substrates, cofactors and chromogens. Other components of a kit can easily be determined by one of skill in the art. Such components may include coating reagents, polyclonal or monoclonal capture antibodies specific for a HR1-drived polypeptide of the disclosure, or a cocktail of two or more of the antibodies, purified or semi-purified extracts of these antigens as standards, MAb detector antibodies, an anti-mouse or anti-human antibody with indicator molecule conjugated thereto, an ELISA plate prepared for absorption, indicator charts for colorimetric comparisons, disposable gloves, decontamination instructions, applicator sticks or containers, a sample preparatory cup, etc. In one embodiment, a kit comprises buffers or other reagents appropriate for constituting a reaction medium allowing the formation of a peptide-antibody complex. Such kits provide a convenient, efficient way for a clinical laboratory to diagnose infection by a SARS-COV-2 virus.

In some embodiments, the diagnostic methods and kits described herein may be used for detecting generally the presence of anti-SARS-COV-2 spike protein antibodies in a sample previously collected from an individual.

In some embodiments, diagnostic methods and kits described herein make use of only one polypeptide selected from the polypeptides belonging to the family of HR1-derived polypeptides of the present disclosure.

In other embodiments, diagnostic methods and kits described herein make use of a plurality of HR1-derived polypeptides selected from the polypeptides belonging to the family of HR1-derived polypeptides of the present disclosure.

### Screening methods

The present disclosure also relates to methods for the screening of compounds that may be subsequently used for preventing or treating an infection of a subject with a SARS-COV-2 virus or for preventing or treating a disease caused by the infection of a subject with a SARS-COV-2 virus.

This disclosure pertains to an *in vitro* method for the screening of compounds useful for preventing a disease caused by the infection of a subject with a SARS-COV-2 virus comprising the steps of :
a) bringing into contact cultured cells in which NKp44L membrane expression is inducible with a candidate compound,
b) adding a HR1-derived peptide of the disclosure to the cultured cells obtained at step a),
c) determining NKp44L membrane expression by the cultured cells obtained at step b), and
d) selecting the candidate compound when NKp44L membrane expression is lower than the NKp44L membrane expression that is determined when step a) is performed in the absence of the said candidate compound.

This disclosure also concerns an *in vitro* method for the screening of compounds useful for treating a disease caused by the infection of a subject with a SARS-COV-2 virus comprising the steps of :
a) bringing into contact cultured cells in which NKp44L membrane expression is inducible with a HR1-derived peptide of the disclosure,
b) adding a candidate compound to the cultured cells obtained at step a),
c) determining NKp44L membrane expression by the cultured cells obtained at step b), and
d) selecting the candidate compound when NKp44L membrane expression is lower than the NKp44L membrane expression that is determined when step a) is performed in the absence of the said candidate compound.

This disclosure also relates to an *in vitro* method for the screening of compounds useful for preventing and/or treating a disease caused by the infection of a subject with a SARS-COV-2 virus comprising the steps of :
a) bringing into contact cultured cells in which NKp44L membrane expression is inducible with (i) a HR1-derived peptide of the disclosure and (ii) a candidate compound,
b) determining NKp44L membrane expression by the cultured cells obtained at step a), and
c) selecting the candidate compound when NKp44L membrane expression is lower than the NKp44L membrane expression that is determined when step a) is performed in the absence of the said candidate compound.

For performing a screening method of the disclosure, any compound of interest, such as a known pharmaceutical active ingredient already known in the art for other medical purpose, may be used.

Cells in which NKp44L membrane expression is inducible may be selected in a group comprising (i) human T cells or a human T cell line, especially a CD3⁺CD8⁺ T cell line or a CD3⁺CD56⁺ T cell line, (ii) lung cell lines endowed with an inducible NKp44L membrane expression such as Calu-3 and (iii) an ACE-2-transfected cell line such as an ACE2-transfected A549 cell line.

Membrane expression of NKp44L may be determined by any known method, especially by using antibodies directed against NLp44L as it is disclosed in the examples herein, which anti-NKp44L antibody may consist of a polyclonal antibody or a monoclonal antibody, such as the monoclonal antibody described by Baychelier et al. (2013 ; Blood Vol 122 : 2935-294).

Membrane expression may be performed by using a labeled secondary antibody.such as a labeled anti-IgM antibody that will allow detection of the binding of the anti-NKp44L antibody when the said anti-NKp44L antibody consists of an IgM antibody, which is the case of the antibody disclosed by Baychelier et al. (2013, *Supra*).

Membrane expression of NKp44L may then be detected and/or quantified for instance by using a known method of flow cytometry, as it is shown in the examples herein. NKp44L expression values may be expressed in arbitrary units, such as in Fluorescence Intensity units.

In a screening method of the present disclosure, a first NKp44L membrane expression value is determined to be "lower" than a second membrane NKp44L expression value when the said first value is of 50% or less than of the said second expression value, preferably 30% or less than of the said second expression value and most preferably 10% or less than of the said second expression value.

In most preferred embodiments of a screening method according to the present disclosure, a candidate compound is selected when, at the last step of the method, it is determined that there is no more NKp44L membrane expression, *i.e.* that NKp44L membrane expression is no more determinable, *i.e.* that NKp44L membrane expression is no more detectable with the determination method which is used.

Amino acid and nucleic acid sequences are found throughout the present description, including in Table 6. In case of any discrepancy between the sequences disclosed in the present specification and an appended sequence listing, *e.g.* a sequence listing according to a WIPO prescribed standard (*e.g.* ST25 or a later version), the correct sequences are deemed being those contained in the present specification.

The present disclosure is further illustrated by the following examples.

### EXAMPLES

### Example 1 : NKp44L expression by PBMC of SARS-COV-2-infected subjects

### A. Materials and Methods

For NKp44L expression by sub-populations of lymphocytes contained in Peripheral Blood Mononuclear cells (PBMC) samples, cell labelling has been performed from 100 µl of a peripheral blood sample according to the three following successive steps :
- Step 1 : labelling with 1 µg/ml of an anti-NKp44L monoclonal antibody during 1h at 4°C, followed by a washing step in a PBS/1%BSA buffer;
- Step 2 : labelling with an anti-IgM-PE antibody (1/500; JacksonLab) during 30 min at 4°C, followed by a washing step in a PBS/1%BSA buffer; [BSA solution from Sigma Aldrich ref: A8412; PBS from Gibco, Ref: 14200-067]
- Step 3 : labelling with a cocktail of anti-CD3-PE-Cya7 (SK7; Ref: 560910), anti-CD4-FITC (RPA-T4; Ref: 561005), anti-CD8-APC (RPA-T8; Ref: 555369) and anti-CD56-PE (B159; ref: 55516) antibodies conjugated to fluorochrome agents [1/100 each from BD pharmingen], followed by a washing step in a PBS/1%BSA buffer and then the cells are resuspended in a volume of 250 µl of PBS buffer.

The suspension of labelled cells are then analysed by flow cytometry (Gallios, Beckman Coulter) and the data were processed by FlowJo^{™}.software.

The anti-NKp44L antibody is described notably in Baychelier et al. (2013, Blood, Vol. 122 : 2935-2942). The use of an anti-NKp44L antibody on human lymphocytes is described notably in Vieillard et al. (2005, Proc Natl Acad Sci, Vol. 102 : 10981-10986).

### B. Results

By flow cytometry, the expression of NKp44L was tested on the surface of the main lymphocyte cell sub-populations from patients infected with Sars-Cov-2.

The lymphocyte sub-populations analyzed by flow cytometry were CD3⁺CD4⁺, CD3⁺CD8⁺, CD3⁺CD56⁺ and CD3⁻CD56⁺.

The 45 patients studied were subdivided into 2 groups: (1) hospitalized patients who have a moderate form of COVID-19 and (2) those admitted to an intensive care unit who have a much more severe form. The data obtained are collated in Table 1.

**Table 1 : NKp44L expression in a plurality of lymphocytes sub-populations of subjects infected by SARS-COV-2**

| | | Healthy donors | Hospitalized patients | Patients in ICU | *P* |
|---|---|---|---|---|---|
| Number | | 20 | 23 | 22 | NS |
| Mean age +/standard deviation | | 39+/-24 | 65+/-20 | 58+/-14 | NS |
| Sex Man/Woman, number | | 10/10 | 12/11 | 7/15 | NS |
| Issue : % patients deceased (number) | | | 8.7 (2) | 36.4 (8) | 0.035 |
| % NKp44L expression : mean +/- standard deviation | CD3+CD4+ | 0.12+/-0.38 | 0.51+/-0.75 | 0.67+/-1.32 | NS |
| | CD3+CD8+ | 0.09+/-1.4 | 3.24+/-0.75 | 2.45+/-2.11 | NS |
| | CD3+CD56+ | 0.21+/-0.17 | 4.46+/-3.75 | 4.02+/-2.25 | NS |
| | CD3-CD56+ | 0.10+/-0.24 | 0.97+/-0.94 | 1.16+/-2.23 | NS |

The statistical values (*P*) were determined according to the test of Mann-Whitney, excepted for the data relating to Sex and Issue, the latter being evaluated with the test of Fisher; "NS" means Non Significant.

Further, preliminary data show that NKp44L is expressed both by the virus-infected cells as well as by bystander cells.

Altogether, the results of Example 1 show that NKp44L expression is induced in a plurality of lymphocyte sub-populations, and especially in T CD3⁺CD8⁺ and T CD3⁺CD56⁺ lymphocytes.

However, according to the presented data, the level of NKp44L expression does seem to be related to the severity of the disease in SARS-COV-2-infected subjects.

### Example 2 : Induction of NKp44L membrane expression by SARS-COV-2 virus particles.

### A. Materials and Methods

### A.1. Obtaining and Generating Biological Materials

### a) Obtaining cell lines

- Vero cells (ATCC - Ref n° CRL1586).
- Human lung cells: Calu-3 (ATCC - Ref n°HTB55) and A549: hu ACA-2 (Creative Biogene Technology - Ref n° CSC-RO0642).

### b) Generation of an isolate of Sars-Cov-2

- Infection of Vero cells with BAL of a patient infected with Sar-Cov-2; SARS-CoV-2 clinical isolate was obtained from BAL of a symptomatic infected patient (#SARS-CoV-2/PSL2020) at Pitié-Salpêtrière hospital, Paris (France). BAL (0.5 mL) was mixed with an equal volume of DMEM without FBS, supplemented with 25 mM Hepes, double concentration of penicillin-streptomycin and miconazole (Sigma), and added to 80% confluent Vero-E6 cells monolayer seeded into a 25 cm² tissue culture flask. After 1 h adsorption at 37°C, 3 mL of infectious media (DMEM supplemented with 2% FBS, penicillin-streptomycin and miconazole) were added. Twenty-four hours post-infection another 2 mL of infectious media were added. Five days post-infection, supernatants were collected, aliquoted and stored at -80°C (P#1). For secondary virus stock, Vero-E6 cells seeded into 25 cm² tissue culture flasks were infected with 0.5 mL of P#1 stored aliquot, and cell-culture supernatant were collected 48 h post-infection and stored at -80°C (P#2).
- Validation of infection after several cell passages of the isolate: SARS-CoV-2 / PSL2020 (Karoyan et al, Comm Biol 2021; 4:197);
- Determination of the viral titer; Infectious viral particles were measured by a standard plaque assay previously described with fixation of cells 72 h post infection (Mendoza EJ, et al. Curr Protoc Microbiol. 2020 Jun;57(1):ecpmc105. doi: 10.1002/cpmc.105).
- Storage at -80 ° C in aliquots of 1 mL.

### A.2. Induction of NKp44L membrane expression by SARS-COV-2

### a) Induction of NKp44L by Sars-Cov-2 particles (infectious or inactivated)

Inactivation of Sars-Cov-2 was performed following the procedure published by Heilingloh et al ("Susceptibility of SARS-CoV-2 to UV irradiation", 2020, Am J Infect Control, Vol. 48(10) : 1273-1275).

Briefly, 600 µL of the virus stock was irradiated for 15 min in a 24 well plate. The plate was placed at a distance of 3 cm from the UV source.

Inactivation of the virus was confirmed after infection of Vero cells.

For practical reasons on the one hand and physiological reasons on the other hand, it was chosen to use, in the following experiments, cells from human lung cell lines, that are known to be targets of Sars-Cov-2, instead of primary lymphocytes from healthy donors or SARS-COV-2-infected subjects.

### B. Results

By flow cytometry, the expression of NKp44L was tested on the surface of cells of the Calu-3 lung line in the presence of viruses that are infectious or inactivated by UV treatment. The methodology used for the labeling is identical to the first two steps of that presented in the Materials and Methods section of Example 1 The data obtained in 3 independent experiments are grouped together in Table 2.

**Table 2 : NKp44L membrane expression by Calu-3 cells in the presence of SARS-COV-2 viral particles**

| | NKp44L membrane expression (geo MFI) | | |
|---|---|---|---|
| | NT | +COV-2 | +i-COV-2 |
| Experiment n° 1 | 112 | 1013 | N/a |
| Experiment n° 2 | 126 | 1540 | 914 |
| Experiment n° 3 | 171 | 1201 | 789 |

MFI: Mean of Fluorescence Intensity; NT : Not Treated; +COV-2 : infection of cells by 5 x 10⁶ TCID₅₀/mL of SARS-COV-2/PSL2020; +i-COV-2 : Treatment of cells with 5 x 10⁶ UV-inactivated SARS-COV-2/PSL2020; TCID50 : mean Tissue Culture Infectious Dose.

These data show that the expression of NKp44L can be induced on the surface of Calu-3 cells in the presence of infectious or UV-inactivated Sars-Cov-2 viral particles.

These data also show that the induction of NKp44L is directly associated with the presence of viral particles.

These data also allow considering that a particular pattern of the Sars-Cov-2 envelope could be involved in the induction of NKp44L (Go / no-Go).

### Example 3 : Induction of NKp44L membrane expression by a specific peptide derived from the SARS-COV-2 spike protein.

### A. Materials and Methods

### A.1. Generation of the Spike overlapping peptide library

- A library of 315 15-mer peptides overlapping 10 amino acids comprising the entire Spike consensus sequence was designed and then synthesized by GeneCust Company (Boynes - France)
- Conditions: Purity:> 85%; Quantity: 1 to 2 mg per peptide.
- Each peptide was individually resuspended at 10 mg / mL then frozen at -80 ° C;
- Generation of 11 super-pools: 10 pools of 30 peptides (pools 1 to 10) and 1 pool of 15 peptides (pool 11).

### A.2. Methodology

The experimental protocol used is as it follows :
- Culture of Calu-3 or A549 cells: hu ACA-2 in a 12-well plate with 1 or 2 × 105 cells per well, respectively.
- 24h stimulation of the cells with 1 ug / mL of each super-pool; - Analysis of the level of expression of NKp44L on the surface of cells.

### B. Results

### B.1. Identification of a relevant pool of SARS-COV-2 spike protein-derived peptides

By flow cytometry, the expression of NKp44L was tested on the surface of the cells of the lung cell lines Calu-3 and A549: hu ACE-2 after 24 hours stimulation of the cells with 1 µg of each super-pool of Spike peptides.

The data obtained in 3 independent experiments for each cell line are depicted in Table 3 at the end of the present description.

The experiments were repeated three times with each of the 2 lung lines studied. The results are similar in each of the experiments. Only the induction of cells by super-pool No. 8 can significantly induce the expression of NKp44L.

### B.2. Identification of a SARS-COV-2 spike protein-derived peptide responsible of the induction of NKp44L membrane expression.

First, super-pool No. 8 comprising 30 peptides, comprised between Spi211 and Spi239 (appendix 1) were sub-divided into 3 mini-pools (8.1, 8.2 and 8.3) of 10 peptides. Each mini-pool was analyzed according to the strategy described above. As a control, an irrelevant pool of 10 peptides was also tested (4.1: Spi91-Spi100) (Appendix 1). The data obtained in 3 independent experiments for each cell line are depiceted in Table 4.

**Table 4 : Nkp44L membrane expression induction in the presence of various of SARS-COV-2 spike protein-derived peptide sub-pools contained in peptide pool n°8**

| Expriment | | | | | |
|---|---|---|---|---|---|
| | Pool n°8 | Negative Control | 8.1 | 8.2 | 8.3 |
| Calu-3 | | | | | |
| n° 1 | | 99 | 117 | 1211 | 924 |
| n° 2 | | 111 | 123 | 1076 | 1002 |
| n°3 | | 109 | 115 | 1098 | 981 |

| A549 | | | | | |
|---|---|---|---|---|---|
| n° 1 | | 82 | 77 | 802 | 756 |
| n°2 | | 81 | 80 | 912 | 811 |
| n°3 | | 71 | 92 | 839 | 738 |

| | | | | | |
|---|---|---|---|---|---|
| MFI: Mean Flurorescence Intensity | | | | | |

The data obtained suggests that one or more peptides contained in the mini-pools of peptides 8.2 and 8.3 can induce the expression of NKp44L.

The relevant SARS-COV-2 spike protein-derived peptide motif could therefore be located between the last peptides of mini-pool 8.2 (Spi-221-Spi230 and the first peptides of 8.2 (Spi-231-Spi239).

To confirm these data, we tested separately:
- 1 pool comprising the first 5 peptides of 8.2 (Spi221-Spi225)
- Individually the last 5 peptides of 8.2 (Spi226, Spi227, Spi228, Spi229 and Spi230)
- Individually the first 5 peptides of 8.3 (Spi231, Spi232, Spi233, Spi234 and Spi235)
- 1 pool comprising the last 5 peptides of 8.3 (Spi236-Spi239)

Each individual pool or peptide was then analyzed according to the methodology previously described. The data obtained in three independent experiments for each of the two cell lines are depicted in Table 5 at the end of the present description.

Our data indicate that 4 overlapping peptides induce the expression of NKp44L in the two lung cell lines tested: the peptides Spi227, Spi229, Spi230 and Spi231.

From the sequences of these four peptides, a pattern of 42 amino acids is deduced, the sequence of which is given below:
N-ter-qmayrfngigvtqnvlyenqklianqfnsaigkiqdslssta-C-ter (SEQ ID NO. 8)

### Example 4 : Analysis of the SARS-COV-2 spike protein-derived peptide inducing NKp44L membrane expression

The various analyzes perfomed in Example 3 revealed a peptide motif of 42 amino acids which is capable of inducing the expression of NKp44L on the surface of the two lung cell lines tested. The study of its amino acid sequence has shown that this motif is located in the HR-2 domain of the S2sub-unit of the SARS-COV-2 spike protein between the amino acid in position 901 and the amino acid in position 942.

Through *in-silico* studies carried out from data which are currently available in databases, it is shown:

### 1- Pattern specificity:

The search in http://www.uniprot.org/peptidesearch/ (UniProtKB 2020_06 results) shows that the peptide pattern is only present in 2266 sequences, including 2252 SARs-Cov-2 sequences, 3 sequences of Bat SARS coronavirus (HKU3-7, HKU3-8 and RaTG13) and 9 sequences of Pangolin coronavirus.

These data clearly indicate that this amino acid sequence is very specific from SARS-COV-2.

### 2- Conservation of the amino acid motif:

Analysis of more than 50 sequences published in https://www.ncbi.nlm.nih.gov/protein/?term=Severe+acute+respiratory+syndrome+corona virus+2+envelope+protein failed to demonstrate mutations in the 42 amino acid motif. In addition, the systematic analysis of the three most notable variants, identified from their reporting origin, namely the United Kingdom (20I/ 501Y.V1 or B.1.1.7), South Africa (20H /501Y.V2 or B.1.351) and Brazil (20J / 501Y.V3 or B.1.1.28), suggests that the mutations that have been identified (including ΔH69 / V70, N501Y, K417N, E484K) are not present in the region between amino acids 901 and 942 where the pattern of 42 amino acids is located.

These data strongly show that this amino acid sequence is highly conserved. This could in part be explained by its location in the HR-1 domain (structural domain of S2 sub-unit), the integrity of which is required for conformational changes that precede the fusion (infection) step.

The first results show that NKp44L is induced on the surface of different cell types by SARS-COV-2 virus particles, including at the membrane of certain lymphocyte and pulmonary cell lines from patients infected with SARS-COV-2 or cells infected *in vitro.* Induction of NKp44L is also achieved in the presence of inactivated viral particles. Finally, preliminary data obtained with two cell lines of pulmonary origin also indicate that a specific and conserved peptide motif of 42 amino acids of the Spike protein is capable of inducing the expression of NKp44L.

**.Table 3 : Nkp44L membrane expression induction in the presence of various pools of SARS-COV-2 spike protein-derived peptides**

| **Exp.** | | **NKp44L membrane expression (Geo MFI)** | | | | | | | | | | | |
|---|---|---|---|---|---|---|---|---|---|---|---|---|---|
| | **# Super Pool** | **Negative Control** | **1** | **2** | **3*** | **4** | **5** | **6** | **7** | **8** | **9** | **10** | **11** |
| **Calu-3** | | | | | | | | | | | | | |
| n°1 | | 117 | 118 | 134 | 225 | 157 | 148 | 152 | 119 | 817 | 132 | 165 | 148 |
| n°2 | | 143 | 174 | 187 | 249 | 129 | 183 | 199 | 201 | 1212 | 179 | 154 | 138 |
| n°3 | | 115 | 109 | 122 | 301 | 132 | 146 | 177 | 191 | 1032 | 135 | 142 | 118 |
| | | | | | | | | | | | | | |

| **A549** | | | | | | | | | | | | | |
|---|---|---|---|---|---|---|---|---|---|---|---|---|---|
| n°1 | | 66 | 86 | 89 | 256 | 67 | 67 | 78 | 98 | 628 | 83 | 81 | 70 |
| n°2 | | 69 | 70 | 75 | 211 | 66 | 69 | 80 | 83 | 649 | 78 | 66 | 67 |

| | | | | | | | | | | | | | |
|---|---|---|---|---|---|---|---|---|---|---|---|---|---|
| Mfi : Mean of Fluorescence Intensity; Negative control: cells were treated only with the secondary fluorescent antibody. 3* : peptide pool 3 exerted a strong cell toxicity. | | | | | | | | | | | | | |

**Table 5 : Nkp44L membrane expression induction in the presence of various of SARS-COV-2 spike protein-derived individual peptides contained in sub-pools n° 8.2 and n° 8.3**

| **Experiment** | **Induction of NKp44L membrane expression** | | | | | | | | | | | | |
|---|---|---|---|---|---|---|---|---|---|---|---|---|---|
| | #Spi | 221-225 | 226 | 227 | 228 | 229 | 230 | 231 | 232 | 233 | 234 | 235 | 236-239 |
| Calu-3 | | | | | | | | | | | | | |
| n° 1 | | 92 | 103 | 2507 | 91 | 2103 | 2213 | 2250 | 421 | 87 | 72 | 76 | 101 |
| n° 2 | | 95 | 112 | 2726 | 103 | 1889 | 2403 | 2406 | 301 | 92 | 80 | 78 | 112 |
| n° 3 | | 103 | 119 | 2592 | 117 | 1875 | 2173 | 2207 | 412 | 80 | 86 | 81 | 110 |

| A549 | | | | | | | | | | | | | |
|---|---|---|---|---|---|---|---|---|---|---|---|---|---|
| n° 1 | | 67 | 53 | 1402 | 75 | 1167 | 1202 | 1308 | 101 | 77 | 65 | 49 | 65 |
| n° 2 | | 82 | 59 | 1517 | 82 | 1021 | 1198 | 1232 | 127 | 75 | 64 | 56 | 75 |
| n° 3 | | 92 | 71 | 1538 | 89 | 993 | 1195 | 1199 | 148 | 68 | 69 | 71 | 75 |

**Table 6 : Sequences**

| Name | Type | Sequence | SEQ ID NO |
|---|---|---|---|
| Spike | Peptide | 1  | 9 |
| Concatemer | Peptide | qmayrfngigvtqnvlyenqklianqfnsaigkiqdslssta | 8 |
| Spi226 | Peptide | qmayrfngigvtqnvlye | 7 |
| Spi227 | Peptide | rfngigvtqnvlyenqkl | 1 |
| Spi228 | Peptide | igvtqnvlyenqklianq | 6 |
| Spi229 | Peptide | qnvlyenqklianqfnsa | 2 |
| Spi230 | Peptide | yenqklianqfnsaigki | 3 |
| Spi231 | Peptide | klianqfnsaigkiqdsl | 4 |
| Spi232 | Peptide | nqfnsaigkiqdslssta | 5 |
| | | | |
| Spike | Nucleic acid | 21562                              atgtttgtt tttcttgttt tattgccact agtctctagt 21601 cagtgtgtta atcttacaac cagaactcaa ttaccccctg catacactaa ttctttcaca 21661 cgtggtgttt attaccctga caaagttttc agatcctcag ttttacattc aactcaggac 21721 ttgttcttac ctttcttttc caatgttact tggttccatg ctatacatgt ctctgggacc 21781 aatggtacta agaggtttga taaccctgtc ctaccattta atgatggtgt ttattttgct 21841 tccactgaga agtctaacat aataagaggc tggatttttg gtactacttt agattcgaag 21901 acccagtccc tacttattgt taataacgct actaatgttg ttattaaagt ctgtgaattt 21961 caattttgta atgatccatt tttgggtgtt tattaccaca aaaacaacaa aagttggatg 22021 gaaagtgagt tcagagttta ttctagtgcg aataattgca cttttgaata tgtctctcag 22081 ccttttctta tggaccttga aggaaaacag ggtaatttca aaaatcttag ggaatttgtg 22141 tttaagaata ttgatggtta ttttaaaata tattctaagc acacgcctat taatttagtg 22201 cgtgatctcc ctcagggttt ttcggcttta gaaccattgg tagatttgcc aataggtatt 22261 aacatcacta ggtttcaaac tttacttgct ttacatagaa gttatttgac tcctggtgat 22321 tcttcttcag gttggacagc tggtgctgca gcttattatg tgggttatct tcaacctagg 22381 acttttctat taaaatataa tgaaaatgga accattacag atgctgtaga ctgtgcactt 22441 gaccctctct cagaaacaaa gtgtacgttg aaatccttca ctgtagaaaa aggaatctat 22501 caaacttcta actttagagt ccaaccaaca gaatctattg ttagatttcc taatattaca 22561 aacttgtgcc cttttggtga agtttttaac gccaccagat ttgcatctgt ttatgcttgg 22621 aacaggaaga gaatcagcaa ctgtgttgct gattattctg tcctatataa ttccgcatca 22681 ttttccactt ttaagtgtta tggagtgtct cctactaaat taaatgatct ctgctttact 22741 aatgtctatg cagattcatt tgtaattaga ggtgatgaag tcagacaaat cgctccaggg 22801 caaactggaa agattgctga ttataattat aaattaccag atgattttac aggctgcgtt 22861 atagcttgga attctaacaa tcttgattct aaggttggtg gtaattataa ttacctgtat 22921 agattgttta ggaagtctaa tctcaaacct tttgagagag atatttcaac tgaaatctat 22981 caggccggta gcacaccttg taatggtgtt gaaggtttta attgttactt tcctttacaa 23041 tcatatggtt tccaacccac taatggtgtt ggttaccaac catacagagt agtagtactt 23101 tcttttgaac ttctacatgc accagcaact gtttgtggac ctaaaaagtc tactaatttg 23161 gttaaaaaca aatgtgtcaa tttcaacttc aatggtttaa caggcacagg tgttcttact 23221 gagtctaaca aaaagtttct gcctttccaa caatttggca gagacattgc tgacactact 23281 gatgctgtcc gtgatccaca gacacttgag attcttgaca ttacaccatg ttcttttggt 23341 ggtgtcagtg ttataacacc aggaacaaat acttctaacc aggttgctgt tctttatcag 23401 gatgttaact gcacagaagt ccctgttgct attcatgcag atcaacttac tcctacttgg 23461 cgtgtttatt ctacaggttc taatgttttt caaacacgtg caggctgttt aatagggct 23521 gaacatgtca acaactcata tgagtgtgac atacccattg gtgcaggtat atgcgctagt | 10 |
| | | 23581 tatcagactc agactaattc tcctcggcgg gcacgtagtg tagctagtca atccatcatt 23641 gcctacacta tgtcacttgg tgcagaaaat tcagttgctt actctaataa ctctattgcc 23701 atacccacaa attttactat tagtgttacc acagaaattc taccagtgtc tatgaccaag 23761 acatcagtag attgtacaat gtacatttgt ggtgattcaa ctgaatgcag caatcttttg 23821 ttgcaatatg gcagtttttg tacacaatta aaccgtgctt taactggaat agctgttgaa 23881 caagacaaaa acacccaaga agtttttgca caagtcaaac aaatttacaa aacaccacca 23941 attaaagatt ttggtggttt taatttttca caaatattac cagatccatc aaaaccaagc 24001 aagaggtcat ttattgaaga tctacttttc aacaaagtga cacttgcaga tgctggcttc 24061 atcaaacaat atggtgattg ccttggtgat attgctgcta gagacctcat ttgtgcacaa 24121 aagtttaacg gccttactgt tttgccacct ttgctcacag atgaaatgat tgctcaatac 24181 acttctgcac tgttagcggg tacaatcact tctggttgga cctttggtgc aggtgctgca 24241 ttacaaatac catttgctat gcaaatggct tataggttta atggtattgg agttacacag 24301 aatgttctct atgagaacca aaaattgatt gccaaccaat ttaatagtgc tattggcaaa 24361 attcaagact cactttcttc cacagcaagt gcacttggaa aacttcaaga tgtggtcaac 24421 caaaatgcac aagctttaaa cacgcttgtt aaacaactta gctccaattt tggtgcaatt 24481 tcaagtgttt taaatgatat cctttcacgt cttgacaaag ttgaggctga agtgcaaatt 24541 gataggttga tcacaggcag acttcaaagt ttgcagacat atgtgactca acaattaatt 24601 agagctgcag aaatcagagc ttctgctaat cttgctgcta ctaaaatgtc agagtgtgta 24661 cttggacaat caaaaagagt tgatttttgt ggaaagggct atcatcttat gtccttccct 24721 cagtcagcac ctcatggtgt agtcttcttg catgtgactt atgtccctgc acaagaaaag 24781 aacttcacaa ctgctcctgc catttgtcat gatggaaaag cacactttcc tcgtgaaggt 24841 gtctttgttt caaatggcac acactggttt gtaacacaaa ggaattttta tgaaccacaa 24901 atcattacta cagacaacac atttgtgtct ggtaactgtg atgttgtaat aggaattgtc 24961 aacaacacag tttatgatcc tttgcaacct gaattagact cattcaagga ggagttagat 25021 aaatatttta agaatcatac atcaccagat gttgatttag gtgacatctc tggcattaat 25081 gcttcagttg taaacattca aaaagaaatt gaccgcctca atgaggttgc caagaattta 25141 aatgaatctc tcatcgatct ccaagaactt ggaaagtatg agcagtatat aaaatggcca 25201 tggtacattt ggctaggttt tatagctggc ttgattgcca tagtaatggt gacaattatg 25261 ctttgctgta tgaccagttg ctgtagttgt ctcaagggct gttgttcttg tggatcctgc 25321 tgcaaatttg atgaagacga ctctgagcca gtgctcaaag gagtcaaatt acattacaca 25381 taa | |
| Concatemer | Nucleic acid* | caa atg gct tat agg ttt aat ggt att gga gtt aca cag aat gtt ctc tat gag aac Q M A Y R F N G I G V T Q N V L Y E N | 11 |
| | | caa aaa ttg att gcc aac caa ttt aat agt gct att ggc aaa att caa gac tca ctt Q   K   L   I   A   N   Q   F   N   S   A   I   G   K   I   Q   D   S   L tct tcc aca gca S   S   T   A | |
| Spi226 | Nucleic acid* | caa atg gct tat agg ttt aat ggt att gga gtt aca cag aat gtt ctc tat gag Q M A Y R F N G I G V T Q N V L Y E | 12 |
| Spi227 | Nucleic acid* | agg ttt aat ggt att gga gtt aca cag aat gtt ctc tat gag aac caa aaa ttg R F N G I G V T Q N V L Y E N Q K L | 13 |
| Spi228 | Nucleic acid* | att gga gtt aca cag aat gtt ctc tat gag aac caa aaa ttg att gcc aac caa I G V T Q N V L Y E N Q K L I A N Q | 14 |
| Spi229 | Nucleic acid* | cag aat gtt ctc tat gag aac caa aaa ttg att gcc aac caa ttt aat agt gct Q N V L Y E N Q K L I A N Q F N S A | 15 |
| Spi230 | Nucleic acid* | tat gag aac caa aaa ttg att gcc aac caa ttt aat agt gct att ggc aaa att Y E N Q K L I A N Q F N S A I G K I | 16 |
| Spi231 | Nucleic acid* | aaa ttg att gcc aac caa ttt aat agt gct att ggc aaa att caa gac tca ctt K L I A N Q F N S A I G K I Q D S L | 17 |
| Spi232 | Nucleic acid* | aac caa ttt aat agt gct att ggc aaa att caa gac tca ctt tct tcc aca gca N Q F N S A I G K Y Q D S L S S T A | 18 |
| His-Tag | Peptide | HHHHHH | 19 |
| HR1 | Peptide | tqnvlyenqklianqfnsaigkiqdslsstasalgklqdvvnqnaqalntlvkqlssnfgaissvlndilsrldkve | 20 |
| HR1 | Nucleic acid | Tacacagaatgttctctatgagaaccaaaaattgattgccaaccaatttaatagtgctattggcaaaattcaagact Cactttcttccacagcaagtgcacttggaaaacttcaagatgtggtcaaccaaaatgcacaagctttaaacacgctt Gttaaacaacttagctccaattttggtgcaatttcaagtgttttaaatgatatcctttcacgtcttgacaaagttga ggctga | 21 |

| | | | |
|---|---|---|---|
| * The corresponding amino acid sequence is described below the nucleic acid sequence. | | | |

## Claims

1. A HR1-derived polypeptide comprising 10 or more consecutive amino acids of the polypeptide of SEQ ID NO. 8, or a polypeptide derivative thereof.

2. The HR1-derived polypeptide according to claim 1, comprising 10 or more consecutive amino acids of a peptide selected in the group consisting of SEQ ID NO. 1 to SEQ ID NO. 5, or a polypeptide derivative thereof.

3. The HR1-derived polypeptide according to any one of claims 1 and 3, which is selected in the group consisting of SEQ ID NO. 1 to SEQ ID NO. 5 and SEQ ID NO. 8, or a polypeptide derivative thereof.

4. A nucleic acid encoding a HR1-polypeptide according to any one of claims 1 to 3.

5. An expression vector comprising a nucleic acid according to claim 4.

6. A recombinant host cell comprising a nucleic acid according to claim 4.

7. An immunogenic composition comprising a HR1-derived polypeptide according to any one of claims 1 to 3.

8. The immunogenic composition according to claim 7, wherein the HR1-derived polypeptide is covalently linked to a carrier molecule.

9. An immunogenic composition comprising a nucleic acid according to claim 4, the said nucleic acid consisting of a ribonucleic acid.

10. An antibody directed against a HR1-derived polypeptide according to any one of claims 1 to 3.

11. An antibody directed against a HR1-derived polypeptide according to any one of claims 1 to 3 for its use for treating a subject infected with a SARS-COV-2 virus.

12. An antibody directed against a HR1-derived polypeptide according to any one of claims 1 to 3 for its use for reducing the clinical symptoms (signs) of a disease associated with an infection of a subject with a SARS-COV-2 virus.

13. An *in vitro* method for detecting and/or quantifying antibodies directed against a HR1-derived polypeptide according to any one of claims 1 to 3 in a sample, comprising the following steps:
a) bringing into contact a sample to be tested with one or more HR1-derived polypeptide according to any one of claims 1 to 3, and
b) detecting and/or quantifying the formation of complexes between the said HR1-derived polypeptide and antibodies present in the said sample.

14. An *in vitro* method for the screening of compounds useful for preventing and/or treating a disease caused by the infection of a subject with a SARS-COV-2 virus comprising the steps of :
a) bringing into contact cultured cells in which NKp44L membrane expression is inducible with (i) a HR1-derived peptide of the disclosure and (ii) a candidate compound,
b) determining NKp44L membrane expression by the cultured cells obtained at step a), and
c) selecting the candidate compound when NKp44L membrane expression is lower than the NKp44L membrane expression that is determined when step a) is performed in the absence of the said candidate compound.
